# EUROPÄISCHE PATENTSCHRIFT

(11) **EP 0 975 220 B1**
(45) Veröffentlichungstag und Bekanntmachung des Hinweises auf die Patenterteilung: **02.04.2003**
(21) Anmeldenummer: 98922649.3
(22) Anmeldetag: 06.04.1998
(51) Int. Cl.: A01N 43/836, C07D 271/07, C07D 413/12, C07D 413/14, C07D 413/04, C07D 417/12, C07D 417/14

(54) **VERWENDUNG VON SULFONYLOXADIAZOLONEN ALS MIKROBIZIDE**
USE OF SULFONYLOXADIAZOLONES AS MICROBICIDES
UTILISATION DE SULFONYLOXADIAZOLONES COMME MICROBICIDES

(30) Priorität: 18.04.1997 DE 19716259
(43) Veröffentlichungstag der Anmeldung: 02.02.2000
(73) Patentinhaber: Bayer CropScience AG, 40789 Monheim (DE)
(72) Erfinder: ASSMANN, Lutz, D-23701 Eutin (DE); GERDES, Peter, D-52080 Aachen (DE); STENZEL, Klaus, D-40595 Düsseldorf (DE)
(86) Internationale Anmeldenummer: EP9801988
(87) Internationale Veröffentlichungsnummer: WO98047369

(56) Entgegenhaltungen:
- US-A- 3 994 909
- US-A- 4 420 486
- DATABASE WPI Section Ch, Week 8722 Derwent Publications Ltd., London, GB; Class C02, AN 87-154900 XP002073605 -& JP 62 093 283 A (IDEMITSU KOSAN CO LTD)
- Zh. Org. Chim. 1991, Band 27, Seiten 1262 - 1270

## Beschreibung

Die vorliegende Erfindung betrifft die Verwendung von teilweise bekannten Sulfonyloxadiazolonen als Mikrobizide im Pflanzenschutz und im Materialschutz. Außerdem betrifft die Erfindung auch neue Sulfonyloxadiazolone und ein Verfahren zu deren Herstellung.

Es sind bereits bestimmte Sulfonyloxadiazolone, wie z.B. das 4-[(4-Chlorphenyl)-sulfonyl]-3-(2,4,6-trimethylphenyl)-1,2,4-oxadiazol-5(4H)-on, bekannt (vgl. Zh. Org. Khim. 27 (1991), 1262 - 1270). Eine biologische Wirkung dieser Verbindungen ist aber bisher noch nicht beschrieben worden.

Weiterhin werden in der US - A 3 994 909 fungizid wirksame 1,2,4 -Oxadiazolin - 5 - one beschrieben, die am Stickstoffatom in der Nachbarposition zur Carbonyl - Gruppe durch Alkoxycarbonyl oder Alkylaminocarbonyl substituiert sind. Entsprechende Verbindungen, die an der betreffenden Stelle einen Sulfonyl - Rest enthalten, werden aber nicht erwähnt.

Es wurde nun gefunden, daß Sulfonyloxadiazolone der Formel 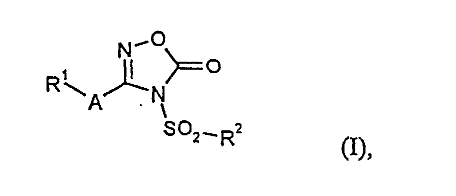 in welcher
- A: für eine Einfachbindung oder für Methandiyl, 1;1-Ethandiyl, 1,2-Ethandiyl 1,1-, 1,2-, 1,3- oder 2,2-Propandiyl, 1,1-, 1,2-, 1,3-, 1,4-, 2,2-, 2,3-Butandiyl, 1,1-, 1,2- oder 1,3-(2-Methyl-propandiyl), 1,1-Ethendiyl, 1,2-Ethendiyl 1,1-, 1,2- oder 1,3-Propendiyl, Ethindiyl, 1,3-Propindiyl oder eine Gruppierung -*O-CH₂-, -*O-CH₂-CH₂-, -*CH₂-O-, -*CH₂-O-CH₂-CH₂-, -*CH₂-S-, -*S-CH₂-, 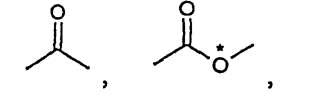 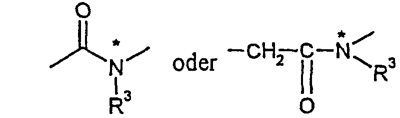 steht, wobei das mit * gekennzeichnete Atom jeweils mit R¹ verbunden ist,
- R³: für Wasserstoff oder Methyl steht,
- R¹: für Wasserstoff, oder für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl steht,
oder
- R¹: für Phenyl steht, das einfach bis dreifach, gleichartig oder verschieden substituiert sein kann durch Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl, gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclphexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch Fluor, Chlor, Brom, Nitro, Methyl, Ethyl, n- oder i-Propyl, n-, i-, s- oder t-Butyl, Methoxy, Ethoxy, n-oder i-Propoxy, Methylthio, Ethylthio, n- oder i-Propylthio, Trifluormethyl, Trifluorethyl, Difluormethoxy, Trifluormethoxy, Difluorchlormethoxy oder Trifluorethoxy,
oder
- R¹: für Thienyl, Furyl oder Pyridyl steht, wobei diese Reste einfach bis dreifach, gleichartig oder verschieden substituiert sein können durch Fluor, Chlor, Brom, Nitro, Amino, Hydroxy, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl und/oder Cyclohexyl,
und/oder durch Phenyl, Phenoxy, Phenylthio, Benzyl, Benzyloxy und/oder Benzylthio, wobei diese aromatischen Reste einfach bis dreifach, gleichartig oder verschieden substituiert sein können durch Fluor, Chlor, Brom, Nitro, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy, Trifluorethoxy, Difluormethylthio, Difluorchlormethylthio und/oder Trifluormethylthio,
und
- R²: für Methyl, Ethyl, n- oder i-Propyl, n-, i-, s-, oder t-Butyl, Dimethylamino oder Diethylamino steht,
oder
- R²: für Phenyl steht, das einfach bis dreifach, gleichartig oder verschieden substituiert sein kann durch Fluor, Chlor, Brom, Nitro, Methyl, Ethyl, Methoxy, Ethoxy, Methylthio, Ethylthio, Trifluormethyl, Trifluorethyl, Difluormethoxy, Trifluormethoxy, Difluorchlormethoxy, Trifluorethoxy, Difluormethylthio, Difluorchlormethylthio oder Trifluormethylthio,
oder
- R²: für Furyl, Thienyl, Oxazolyl, Isoxazolyl, Pyrazolyl, Imidazolyl, Thiazolyl, Isothiazolyl, Oxadiazolyl, Thiadiazolyl, Pyridinyl, Pyridazinyl, Pyrazinyl, 1,2,3-Triazinyl, 1,2,4-Triazinyl, 1,3,5-Triazinyl, 1,2,4-Triazolonyl, Pyrrolidinyl, Piperidinyl oder Morpholinyl steht, wobei diese Reste einfach, zweifach oder dreifach, gleichartig oder verschieden substituiert sein können durch Fluor, Chlor, Brom, Cyano, Nitro, Amino, Hydroxy, Carbamoyl, Methyl, Ethyl, n- oder i-Propyl, n-, i-, s-, oder t-Butyl, Cyclopropyl, Methoxy, Ethoxy, n- oder i-Propoxy, Trifluormethyl, Trifluorethyl, Difluormethoxy, Trifluormethoxy, Acetyl, Propionyl, Methoxycarbonyl, Ethoxycarbonyl, Hydroxyiminomethyl, Hydroxyiminoethyl, Methoxyiminomethyl, Ethoxyiminomethyl, Methoxyiminoethyl und/oder Ethoxyiminoethyl,
sehr gut zur Bekämpfung von unerwünschten Mikroorganismen im Pflanzenschutz und im Materialschutz geeignet sind.

Überraschenderweise zeigen die erfindungsgemäß verwendbaren Sulfonyloxadiazolone der Formel (I) eine wesentlich bessere Wirksamkeit gegen unerwünschte Mikroorganismen, insbesondere Pilze, als die konstitutionell ähnlichsten, vorbekannten Stoffe gleicher Wirkungsrichtung.

Die erfindungsemäß verwendbaren Stoffe sind durch die Formel (I) allgemein definiert.

A steht bevorzugt für eine Einfaehbindung oder fiir Methandiyl, 1,1-Ethandiyl, 1,2-Ethandiyl 1,1-, 1,2-, 1,3-oder 2,2-Propandiyl, 1,1-, 1,2-, 1,3-, 1,4-2,2-, 2,3-Butandiyl, 1,1-, 1,2- oder 1,3-(2-Methyl-propandiyl), 1,2-Ethendiyl, 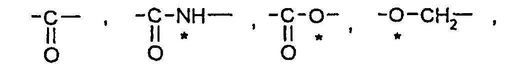 -*O-CH₂-CH₂-, -*CH₂-O-, -*CH₂-O-CH₂-CH₂-, -*S-CH₂-, -*CH₂-S- oder 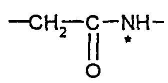 wobei das mit * gekennzeichnete Atom jeweils mit R¹ verbunden ist.
- R¹: steht bevorzugt fiir Wasserstoff oder Cyclohexenyl.
- R¹: steht außerdem bevorzugt fiir gegebenenfalls einfach oder zweifach durch Fluor, Chlor, Brom, Methyl, Ethyl, Methoxy, Nitro, Trifluormethyl, Trifluormethoxy, Phenyl und/oder Phenoxy substituiertes Phenyl.
- R¹: steht außerdem bevorzugt fiir einen Rest der Formel 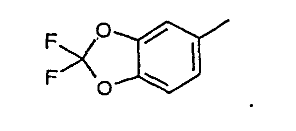
- R¹: steht weiterhin bevorzugt für Thienyl, Furyl oder Pyridyl.
- R²: steht bevorzugt für Methyl, Ethyl, n- oder i-Propyl, n-, i-, s-, oder t-Butyl, Dimethylamino oder Diethylamino.
- R²: steht außerdem bevorzugt für gegebenenfalls einfach oder zweifach durch Fluor, Chlor, Brom, Nitro, Methyl, Ethyl und/oder Methoxy substituiertes Phenyl.
- R²: steht weiterhin bevorzugt für gegebenenfalls einfach, zweifach oder dreifach durch Chlor, Brom, Amino, Hydroxy, Methyl und/oder Cyclopropyl substituiertes, Furyl, Thienyl, Isoxazolyl, Pyrazolyl, Thiazolyl, Imidazolyl, Pyridyl oder 1,2,4-Triazolonyl.
- R²: steht ferner bevorzugt für Pyrrolidinyl, Piperidinyl oder Morpholinyl.

Die erfindungsgemäß verwendbaren Sulfonyloxadiazolone der Formel (I) sind teilweise bekannt (vgl. Zh. Org. Khim. 27 (1991), 1262 - 1270).

Die Sulfonyloxadiazolone der Formel 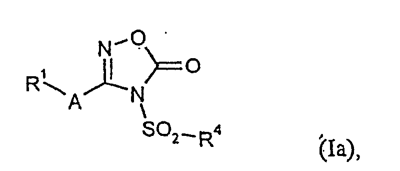 in welcher
- A: fiir eine Einfachbindung oder fiir Methandiyl, 1,1-Ethandiyl, 1,2-Ethandiyl 1,1-, 1,2-, 1,3- oder 2,2-Propandiyl, 1,1-, 1,2-, 1,3-, 1,4-, 2,2-, 2,3-Butandiyl, 1,1-, 1,2- oder 1,3-(2-Methyl-propandiyl), 1,1-Ethendiyl, 1,2-Ethendiyl 1,1-, 1,2- oder 1,3-Propendiyl, Ethindiyl, 1,3-Propindiyl oder eine Gruppierung -*O-CH₂-, -*O-CH₂-CH₂-, -*CH₂-O-, -*CH₂-O-CH₂-CH₂-, -*CH₂-S-, -*S-CH₂-, 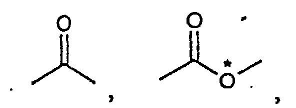 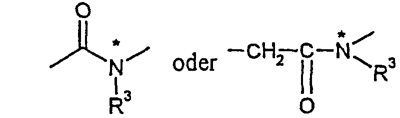 steht, wobei das mit * gekennzeichnete Atom jeweils mit R¹ verbunden ist,
- R³: für Wasserstoff oder Methyl steht,
- R¹: für Wasserstoff, oder für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl steht,
oder
- R¹: für Phenyl steht, das einfach bis dreifach, gleichartig oder verschieden substituiert sein kann durch Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoxirninomethyl, Ethoximinomethyl, Methoximinoethyl oder Ethoximinoethyl,
gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch Fluor, Chlor, Brom, Nitro, Methyl, Ethyl, n- oder i-Propyl, n-, i-, s- oder t-Butyl, Methoxy, Ethoxy, n-oder i-Propoxy, Methylthio, Ethylthio, n- oder i-Propylthio, Trifluormethyl, Trifluorethyl, Difluormethoxy, Trifluormethoxy, Difluorchlormethoxy oder Trifluorethoxy,
oder
- R¹: für Thienyl, Furyl oder Pyridyl steht, wobei diese Reste einfach bis dreifach, gleichartig oder verschieden substituiert sein können durch Fluor, Chlor, Brom, Nitro, Amino, Hydroxy, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl und/oder Cyclohexyl,
und/oder durch
Phenyl, Phenoxy, Phenylthio, Benzyl, Benzyloxy und/oder Benzylthio, wobei diese aromatischen Reste einfach bis dreifach, gleichartig oder verschieden substituiert sein können durch Fluor, Chlor, Brom, Nitro, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy, Trifluorethoxy, Difluormethylthio, Difluorchlormethylthio und/oder Trifluormethylthio, und
- R⁴: für Dimethylamino oder Diethylamino steht,
oder
- R⁴: für durch Nitro substituiertes Phenyl steht, das zusätzlich noch einfach oder zweifach, gleichartig oder verschieden substituiert sein kann durch Fluor, Chlor, Brom, Nitro, Methyl, Ethyl, Methoxy, Ethoxy, Methylthio, Ethylthio, Trifluormethyl, Trifluorethyl, Difluormethoxy, Trifluormethoxy, Difluorchlormethoxy, Trifluorethoxy, Difluormethylthio, Difluorchlormethylthio oder Trifluormethylthio,
oder
- R⁴: für Furyl, Thienyl, Oxazolyl, Isoxazolyl, Pyrazolyl, Imidazolyl, Thiazolyl, Isothiazolyl, Oxadiazolyl, Thiadiazolyl, Pyridinyl, Pyridazinyl, Pyrazinyl, 1,2,3-Triazinyl, 1,2,4-Triazinyl, 1,3,5-Triazinyl, 1,2,4-Triazolonyl, Pyrrolidinyl, Piperidinyl oder Morpholinyl steht, wobei
diese Reste einfach, zweifach oder dreifach, gleichartig oder verschieden substituiert sein können durch Fluor, Chlor, Brom, Cyano, Nitro, Amino, Hydroxy, Carbamoyl, Methyl, Ethyl, n- oder i-Propyl, n-, i-, s-, oder t-Butyl, Cyclopropyl, Methoxy, Ethoxy, n- oder i-Propoxy, Trifluormethyl, Trifluorethyl, Difluormethoxy, Trifluormethoxy, Acetyl, Propionyl, Methoxycarbonyl, Ethoxycarbonyl, Hydroxyiminomethyl, Hydroxyiminoethyl, Methoxyiminomethyl, Ethoxyiminomethyl, Methoxyirninoethyl und/oder Ethoxyiminoethyl,
sind neu.

Die Sulfonyloxadiazolone der Formel (Ia) lassen sich herstellen, indem man

Oxadiazolone der Formel 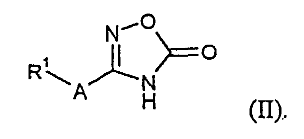 in welcher
- A und R¹: die oben angegebenen Bedeutungen haben,
mit Sulfonsäurehalogeniden der Formel

R⁴-SO₂-X (III)

in welcher
- R⁴: die oben angegebene Bedeutung hat und
- X: für chlor steht,
gegebenenfalls in Gegenwart eines Säurebindemittels und gegebenenfalls in Gegenwart eines Verdünnungsmittels umsetzt.

Die übrigen Verbindungen der Formel (I) lassen sich in gleicher Weise herstellen.

Die neuen Sulfonyloxadiazolone sind durch die Formel (Ia) allgemein definiert. In dieser Formel haben A und R¹ vorzugsweise diejenigen Bedeutungen, die bereits im Zusammenhang mit der Beschreibung der Sulfonyloxadiazolone der Formel (I) vorzugsweise für diese Reste genannt wurden.
- R⁴: steht bevorzugt für Dimethylamino oder Diethylamino,
- R⁴: steht außerdem bevorzugt für durch Nitro substituiertes Phenyl, das noch zusätzlich durch Fluor, Chlor, Brom, Nitro, Methyl, Ethyl oder Methoxy substituiert sein kann.
- R⁴: steht weiterhin bevorzugt für gegebenenfalls einfach, zweifach oder dreifach durch Chlor, Brom, Amino, Hydroxy, Methyl und/oder Cyclopropyl substituiertes, Furyl, Thienyl, Isoxazolyl, Pyrazolyl. Thiazolyl, Imidazolyl, Pyridyl oder 1,2,4-Triazolonyl.
- R⁴: steht ferner bevorzugt für Pyrrolidinyl, Piperidinyl oder Morpholinyl.

Als Beispiele für erfindungsgemäße Stoffe seien die in den folgenden Tabellen aufgeführten Sulfonyloxadiazolone genannt:

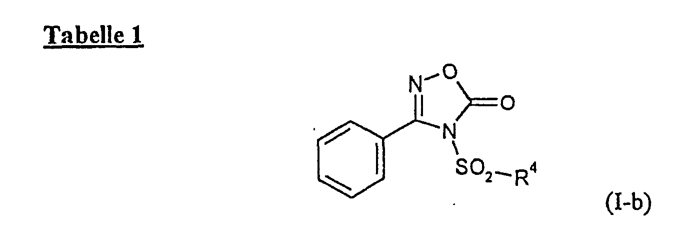

wobei R⁴ für die folgenden Substituenten steht:

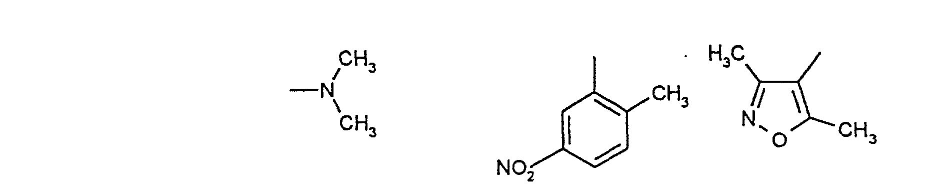

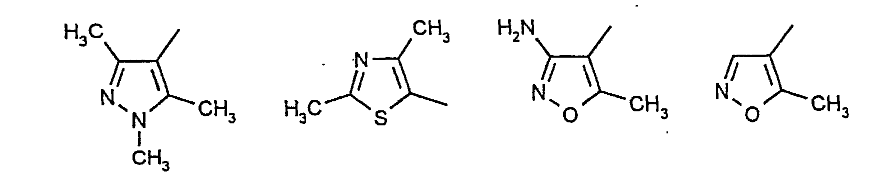

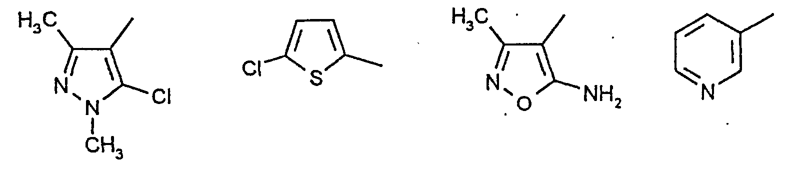

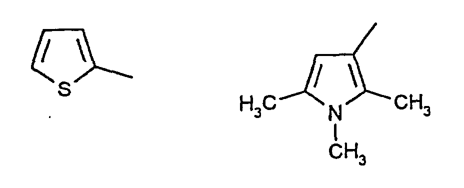

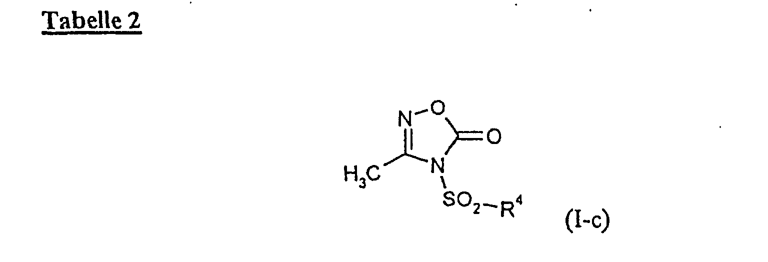

wobei R⁴ für die in Tabelle 1 genannten Substituenten steht.

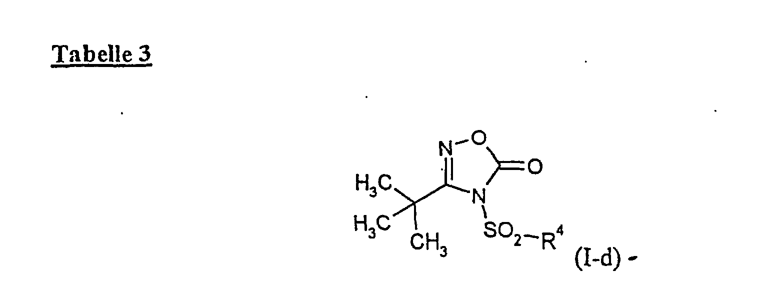

wobei R⁴ für die in Tabelle 1 genannten Substituenten steht.

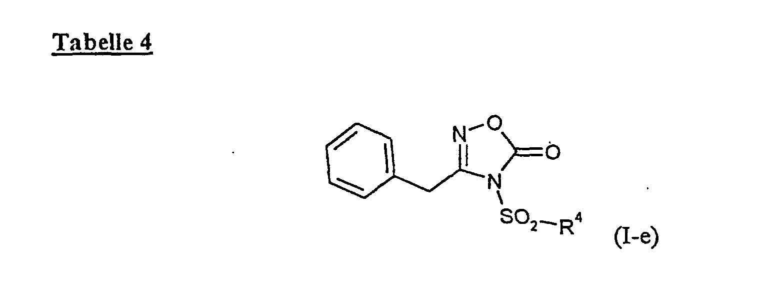

wobei R⁴ für die in Tabelle 1 genannten Substituenten steht.

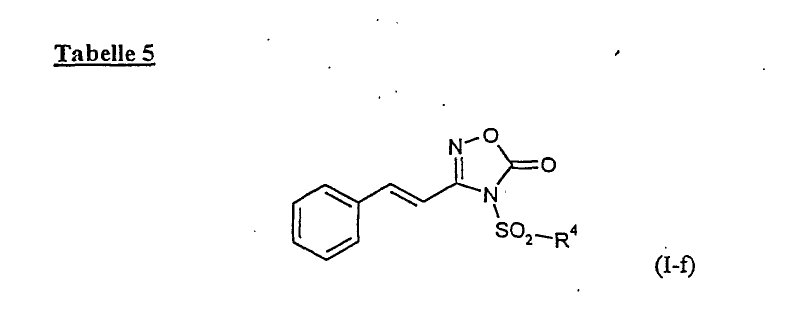

wobei R⁴ für die in Tabelle 1 genannten Substituenten steht.

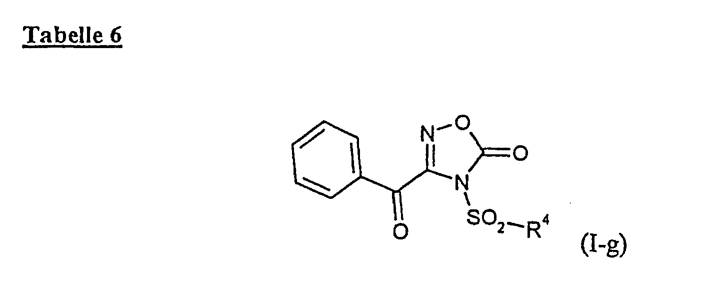

wobei R⁴ für die in Tabelle 1 genannten Substituenten steht.

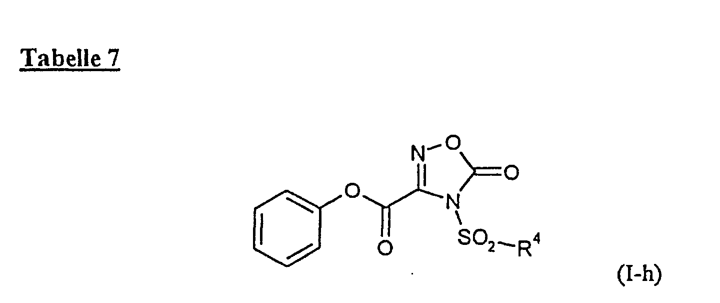

wobei R⁴ für die in Tabelle 1 genannten Substituenten steht.

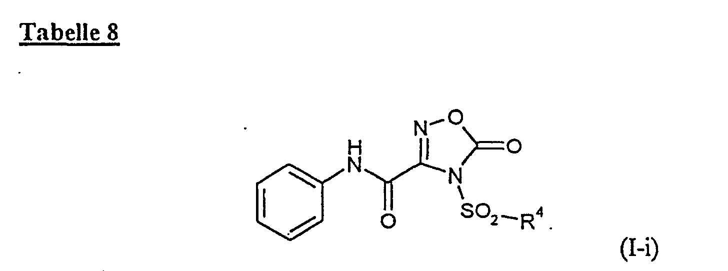

wobei R⁴ für die in Tabelle 1 genannten Substituenten steht.

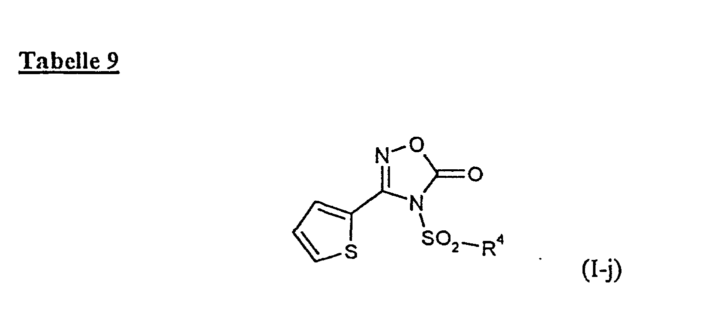

wobei R⁴ für die in Tabelle 1 genannten Substituenten steht.

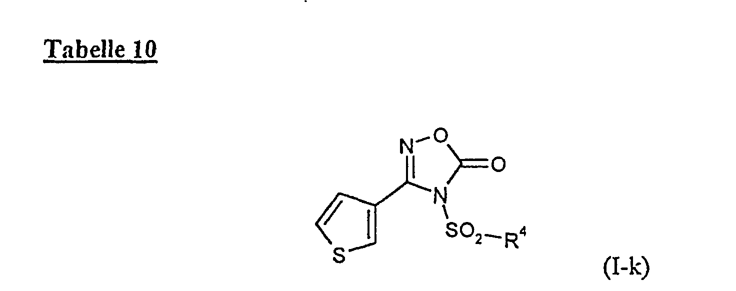

wobei R⁴ für die in Tabelle 1 genannten Substituenten steht.

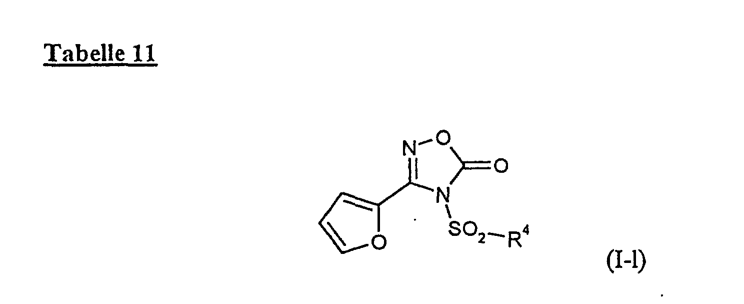

wobei R⁴ für die in Tabelle 1 genannten Substituenten steht.

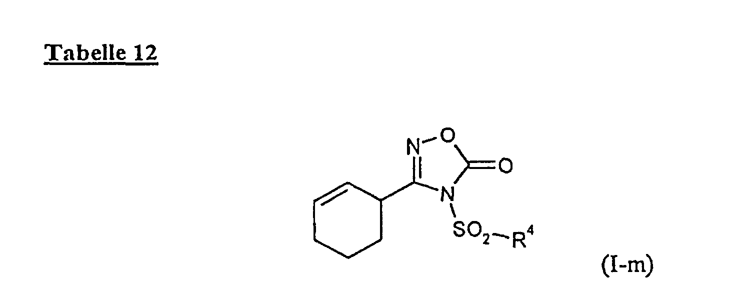

wobei R⁴ für die in Tabelle 1 genannten Substituenten steht.

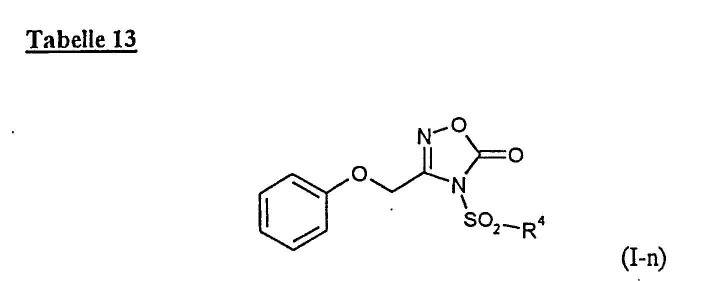

wobei R⁴ für die in Tabelle 1 genannten Substituenten steht.

Verwendet man beispielsweise 3-Phenyl-4H-[1,2,4]oxadiazol-5-on und 3,5-Dimethylisoxazol-4-sulfonylchlorid als Ausgangsstoffe, so läßt sich der Verlauf des erfindungsgemäßen Verfahrens durch das folgende Formelschema veranschaulichen:

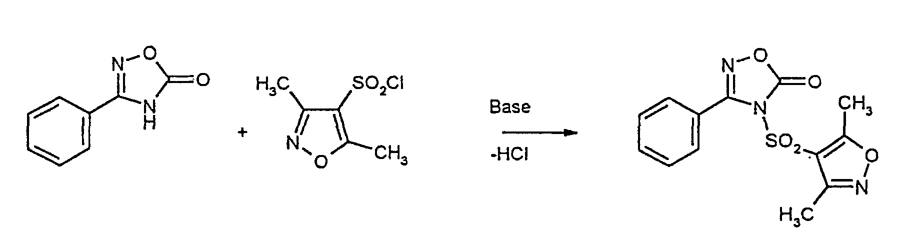

Die zur Durchführung des erfindungsgemäßen Verfahrens als Ausgangstoffe benötigten Oxadiazolone sind durch die Formel (II) allgemein definiert. In dieser Formel haben A und R¹ vorzugsweise diejenigen Bedeutungen, die bereits im Zusammenhang mit der Beschreibung der etfindungsgemäßen Verbindungen der Formel (I) als bevorzugt bevorzugt für A und R¹ angegeben wurden.

Die Oxadiazolone der Formel (II) sind bekannt oder können nach bekannten Methoden hergestellt werden (vgl. Synthesis 6, (1983), 483-486; J. Heterocyclic Chem. 10, (1973), 357-362; J. Org. Chem. 41, (1976), 3233; J. Heterocyclic Chem. 30 (5), (1993), 1253-1260).

Die zur Durchführung des erfindungsgemäßen Verfahrens als Reaktionskomponenten weiterhin benötigten Sulfonsäurehalogenide sind durch die Formel (III) allgemein definiert. In dieser Formel (III) hat R⁴ vorzugsweise diejenige Bedeutung, die bereits im Zusammenhang mit der Beschreibung der erfindungsgemäßen Verbindungen der Formel (I-a) als bevorzugt
für R⁴ angegeben wurde.

Die Sulfonsäurehalogenide der Formel (III) sind bekannt oder können nach bekannten Verfahren hergestellt werden (J. Heterocyclic Chem. 1981, 997-1006).

Als Verdünnungsmittel zur Durchführung des erfindungsgemäßen Verfahrens kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Ester wie Essigsäuremethylester oder Essigsäureethylester.

Als Säurebindemittel kommen bei der Durchführung des erfindungsgemäßen Verfahrens alle üblichen anorganischen oder organischen Basen infrage. Vorzugsweise verwendbar sind Erdalkalimetall- oder Alkalimetallhydride, -hydroxide, -amide, -alkoholate, -acetate, -carbonate oder -hydrogencarbonate, wie Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Nätriumcarbonat, Kaliumcarbonat, Kaliumhydrogencarbonat oder Natriumhydrogencarbonat, ferner Ammonium-Verbindungen, wie Ammoniumhydroxid, Ammoniumacetat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 20°C und 120°C.

Zur Durchführung des erfindungsgemäßen Verfahrens setzt man pro Mol an Oxadiazolon der Formel (II) im allgemeinen 1 bis 2 Mol, vorzugsweise 1 bis 1,3 Mol, an Sulfonsäurehalogenid der Formel (III) und gegebenenfalls 1,0 bis 2,0 Mol, vorzugsweise 1,0 bis 1,3 Mol an Säureakzeptor ein.

Das erfindungsgemäße Verfahren wird im allgemeinen unter Atmosphärendruck durchgeführt. Es ist jedoch auch möglich, unter erhöhtem oder vermindertem Druck - im allgemeinen zwischen 0,1 bar und 10 bar - zu arbeiten.

Die Aufarbeitung erfolgt nach üblichen Methoden.

Die erfindungsgemäß verwendbaren Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimüm;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäß verwendbaren Wirkstoffe mit besonders gutem Erfolg zur Bekämpfung von Krankheiten im Wein-, Obst- und Gemüseanbau, wie beispielsweise gegen Phytophthora- und Plasmopara-Arten, einsetzen. Mit gutem Erfolg werden auch Getreidekrankheiten, wie beispielsweise Leptosphaeria- oder Pyrenophora-Arten, sowie Reiskrankheiten, wie beispielsweise Pyricularia-Arten, bekämpft.

Die erfindungsgemäß verwendbaren Wirkstoffe eignen sich auch zur Steigerung des Ernteertrages. Sie sind außerdem mindertoxisch und weisen eine gute Pflanzenverträglichkeit auf.

Im Materialschutz lassen sich die erfindungsgemäß verwendbaren Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Didobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-lH-1,2,4-triazol-1-ethanol,
(5RS,6RS)-6-Hydroxy-2,2,7,7-tetramethyl-5-(1H-1,2,4-triazol-1-yl)-3-octanon, (E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2, 6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2, 5 -dion,
3, 5-Dichlor-N-[cyan[(1-methyl-2-propynyl)-oxy]-methyl]-benzamid,
3-(1,1-Dimethylpropyl-1-oxo-1H-inden-2-carbonitril,
3 -[2-(4-Chlorphenyl)-5-ethoxy-3 -isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,

Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,

Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin,

Lamda-cyhalothrin, Lufenuron,

Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,

Naled, NC 184, Nitenpyram

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,

Quinalphos,

Salithion, Sebufos, Silafluofen, Sulfotep, Sulprofos,

Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,

Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäß verwendbaren Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die zum Schutz technischer Materialien verwendeten Mittel enthalten die Wirkstoffe im allgemeinen in einer Menge von 1 bis 95 Gewichts-%, bevorzugt von 10 bis 75 Gewichts-%.

Die Anwendungskonzentrationen der erfindungsgemäß verwendbaren Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung von erfindungsgemäß verwendbaren Wirkstoffen werden durch die folgenden Beispiele veranschaulicht.

### Herstellungsbeispiele

### Beispiel 1

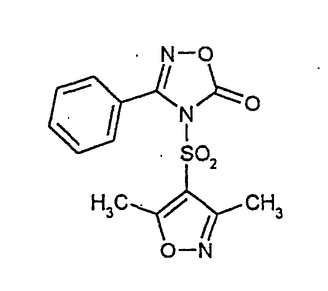

Ein Gemisch aus 2,0 g (15 mmol) 3-Phenyl-4H-[1,2,4]oxadiazol-5-on und 30 ml absolutem Tetrahydrofuran wird bei Raumtemperatur unter Rühren mit 0,40 g (10 mmol) Natriumhydridsuspension (60%ig) versetzt und danach 10 Minuten bei Raumtemperatur gerührt. Anschließend fügt man 1,9 g (10 mmol) 3,5-Dimethylisoxazol-4-sulfonylchlorid hinzu und rührt weitere 20 Stunden bei Raumtemperatur. Zur Aufarbeitung wird das Reaktionsgemisch in 150 ml Wasser gegossen. Das entstehende Gemisch wird zweimal mit je 80 ml Essigsäureethylester extrahiert. Die vereinigten organischen Phasen werden über Natriumsulfat getrocknet und unter vermindertem Druck eingeengt. Der verbleibende Rückstand wird mit Methylenchlorid als Laufmittel an Kieselgel chromatographiert. Man erhält auf diese Weise 0,3 g. (9% der Theorie) an 4-(3,5-Dimethylisoxazol-4-sulfonyl)-3-phenyl-4H-[1,2,4]oxadiazol-5-on in Form eines farblosen Feststoffes vom Schmelzpunkt 90 bis 95°C.

### Beispiel 2

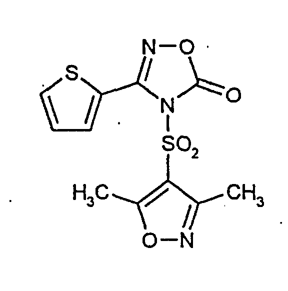

Ein Gemisch aus 1,7 g (10 mmol) 3-Thiophen-2-yl-4H-[1,2,4]oxadiazol-5-on und 30 ml absolutem Acetonitril wird bei Raumtemperatur unter Rühren mit 2,3 g (17 mmol) pulverisiertem Kaliumcarbonat versetzt. Anschließend fügt man 1,0 g (5,5 mmol) 2,0 g (10 mmol) 3,5-Dimethylisoxazol-4-sulfonylchlorid hinzu und rührt weitere 20 Stunden bei Raumtemperatur. Zur Aufarbeitung wird das Reaktionsgemisch in 150 ml Wasser gegossen. Der entstehende Feststoff wird abfiltriert, zuerst mit 10 ml Wasser und anschließend mit 20 ml Diethylether gewaschen und getrocknet. Man erhält 1,77 g (57% der Theorie) 4-(3,5-Dimethylisoxazol-4-sulfonyl)-3-thiophen-2-yl-4H-[1,2,4]oxadiazol-5-on in Form eines gelben Feststoffes vom Schmelzpunkt 138°C.

Nach den zuvor angegebenen Methoden werden auch die in der folgenden Tabelle 14 aufgeführten Sulfonyloxadiazolone der Formel (I) hergestellt.

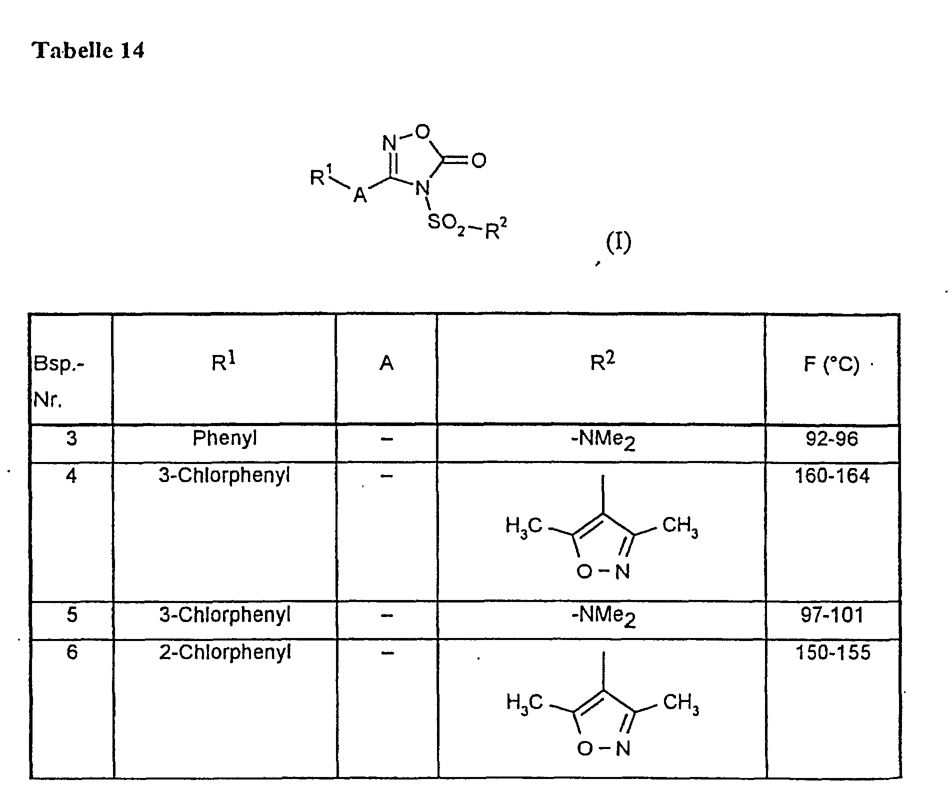

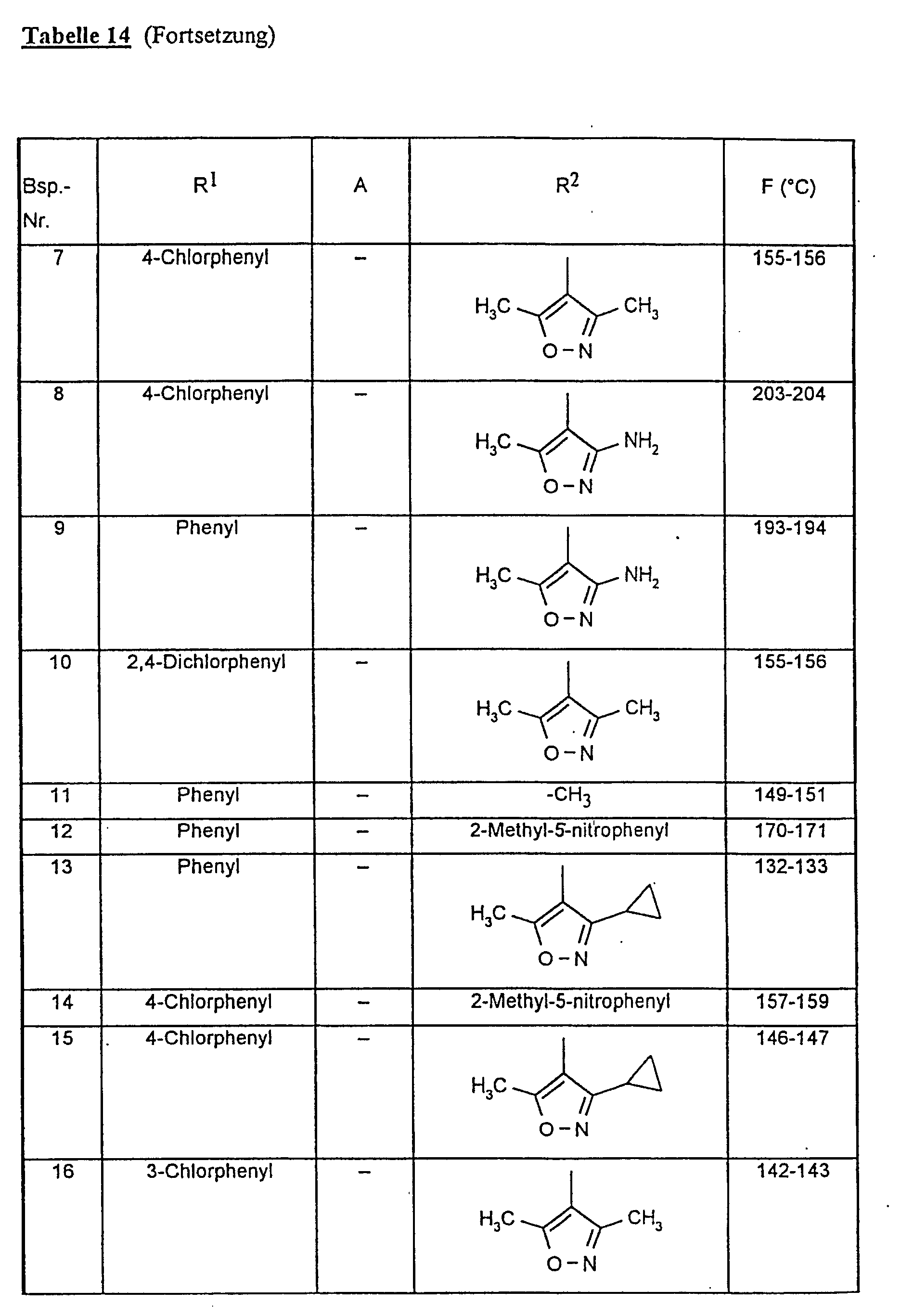

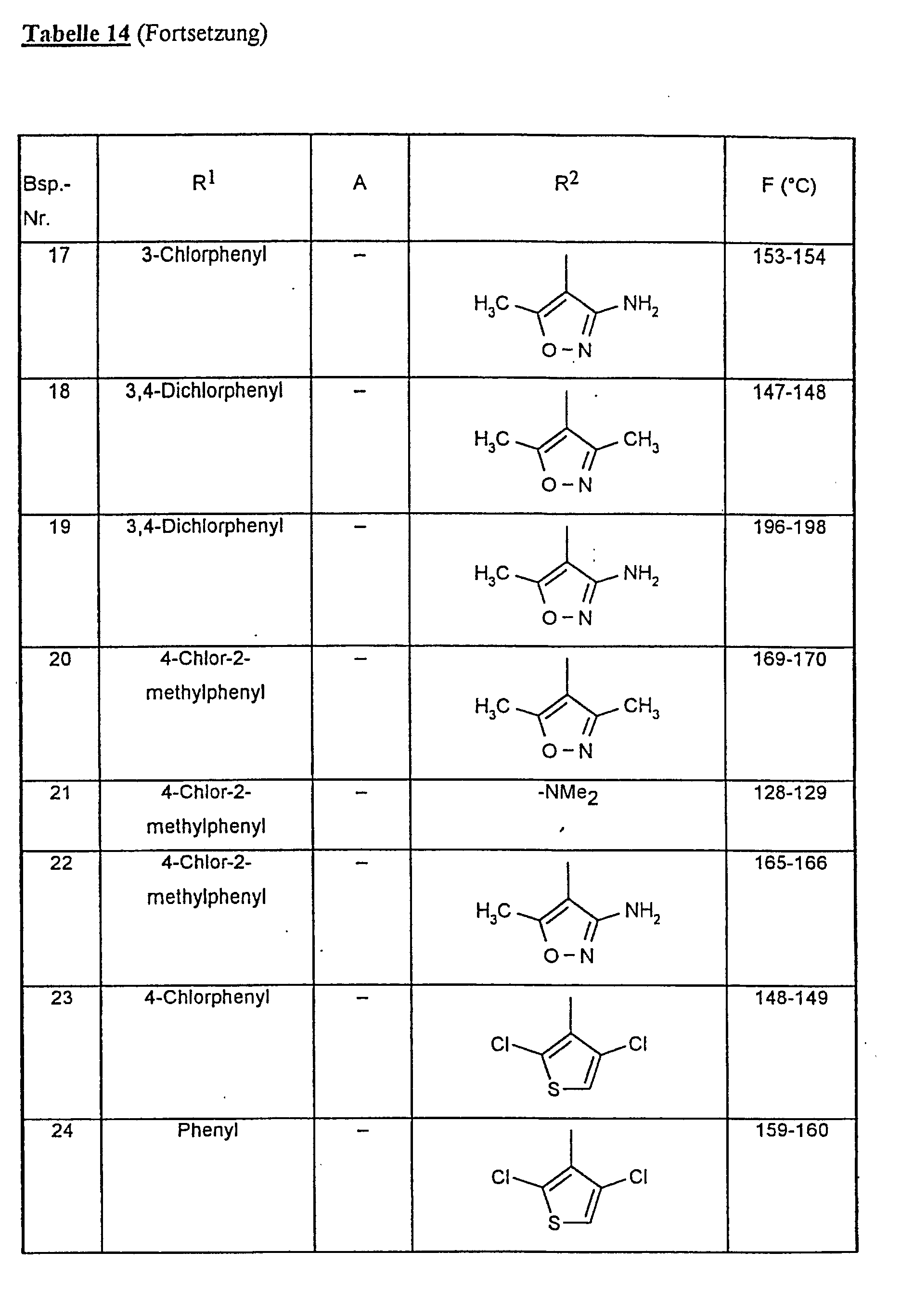

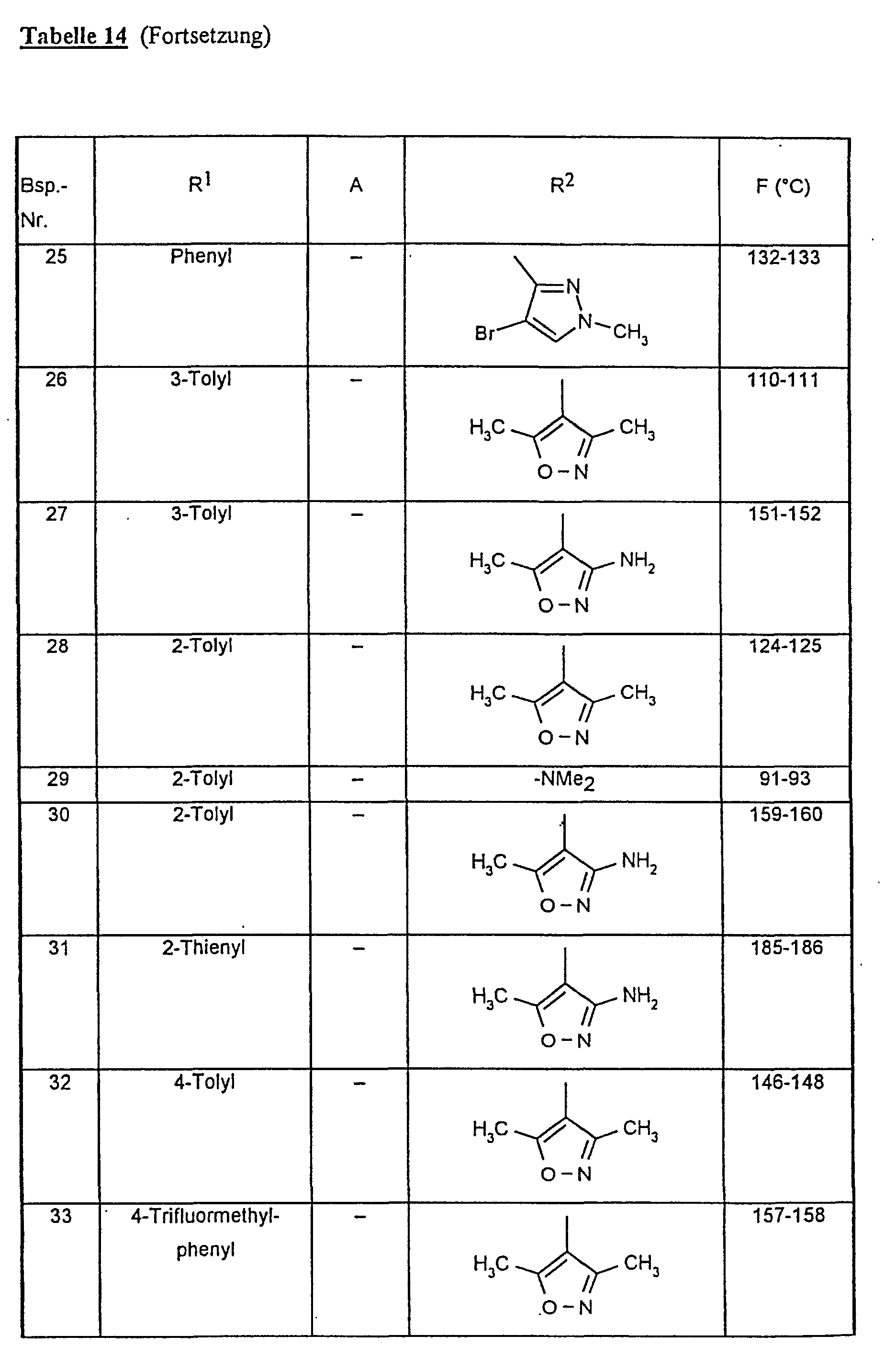

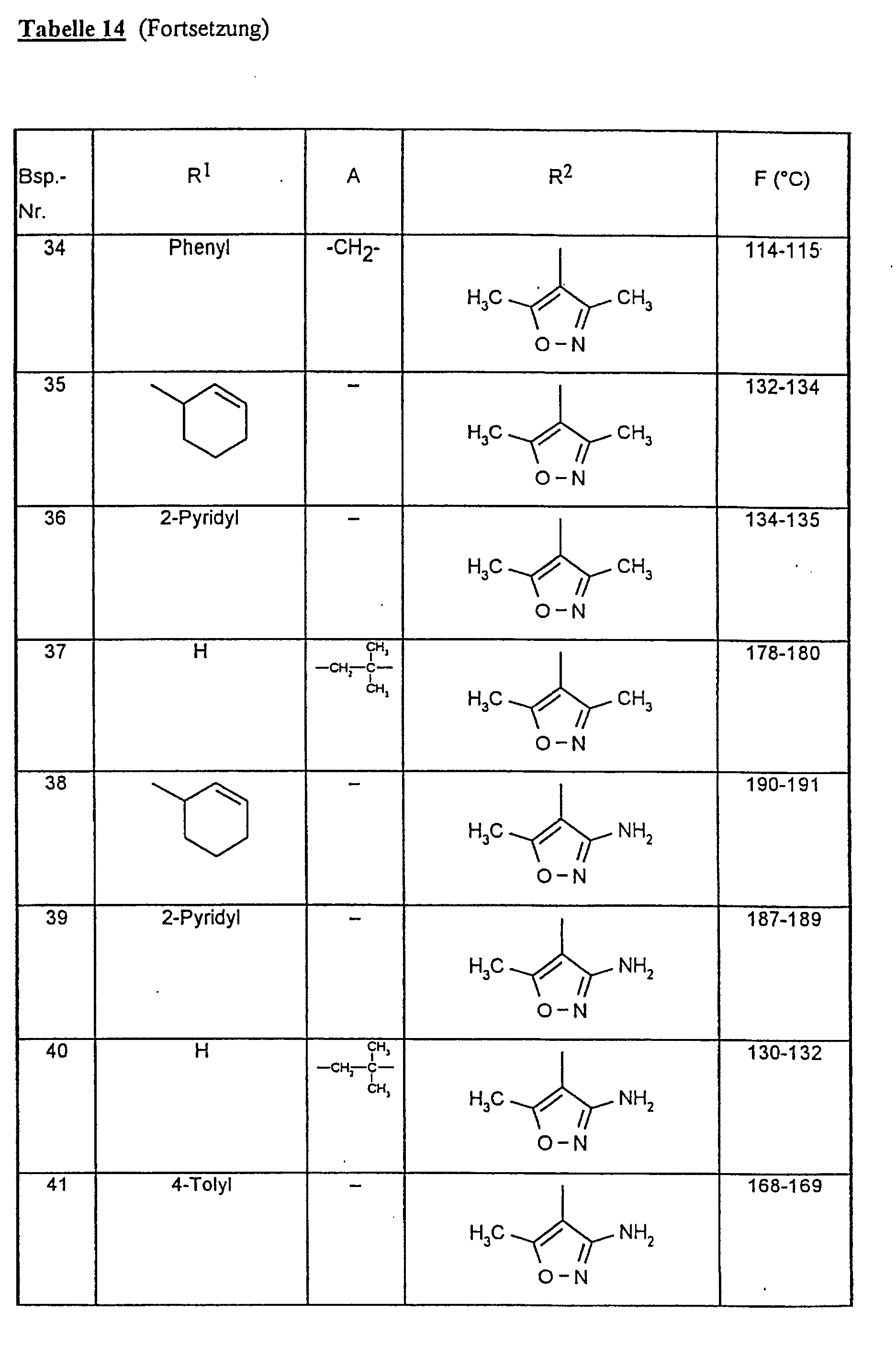

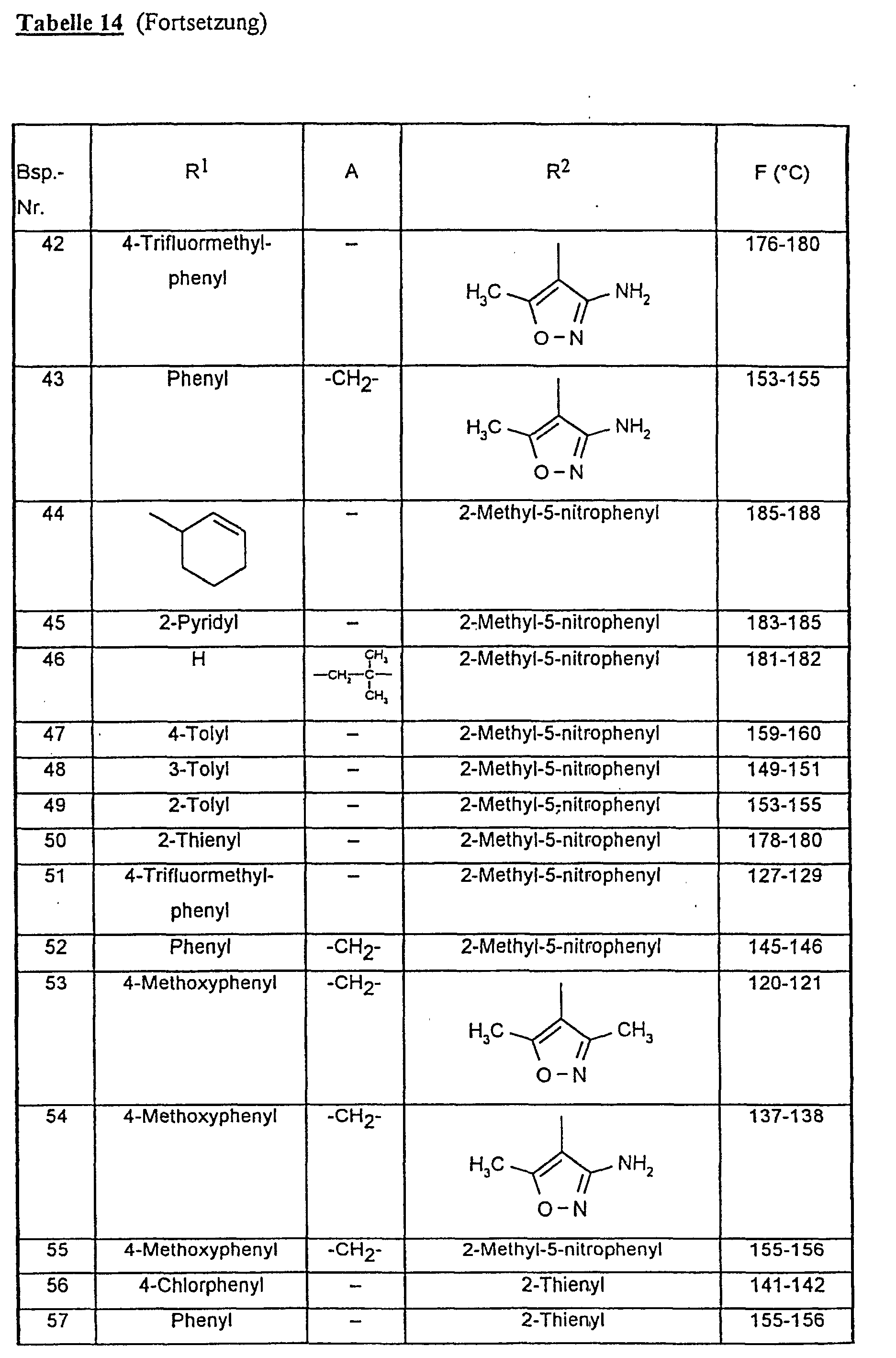

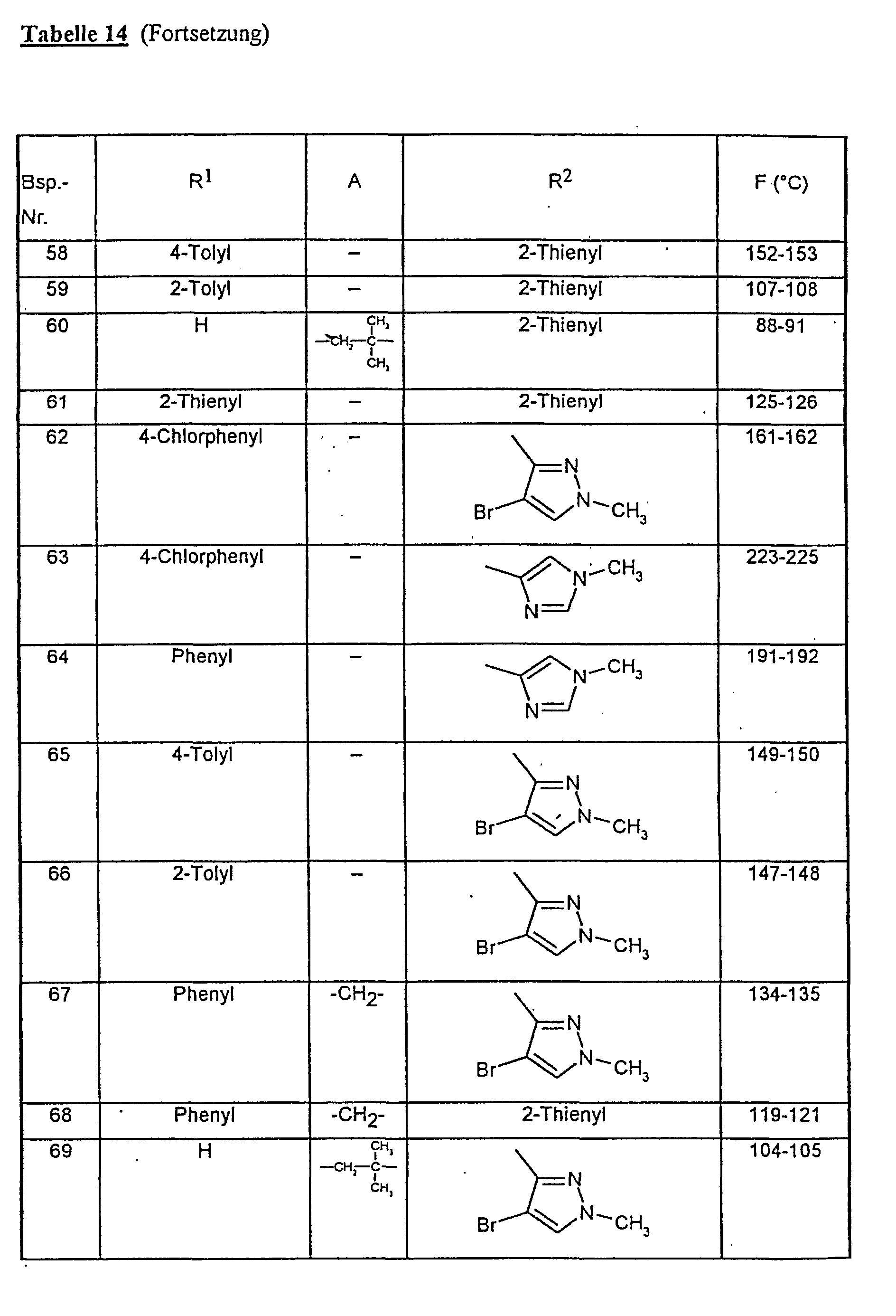

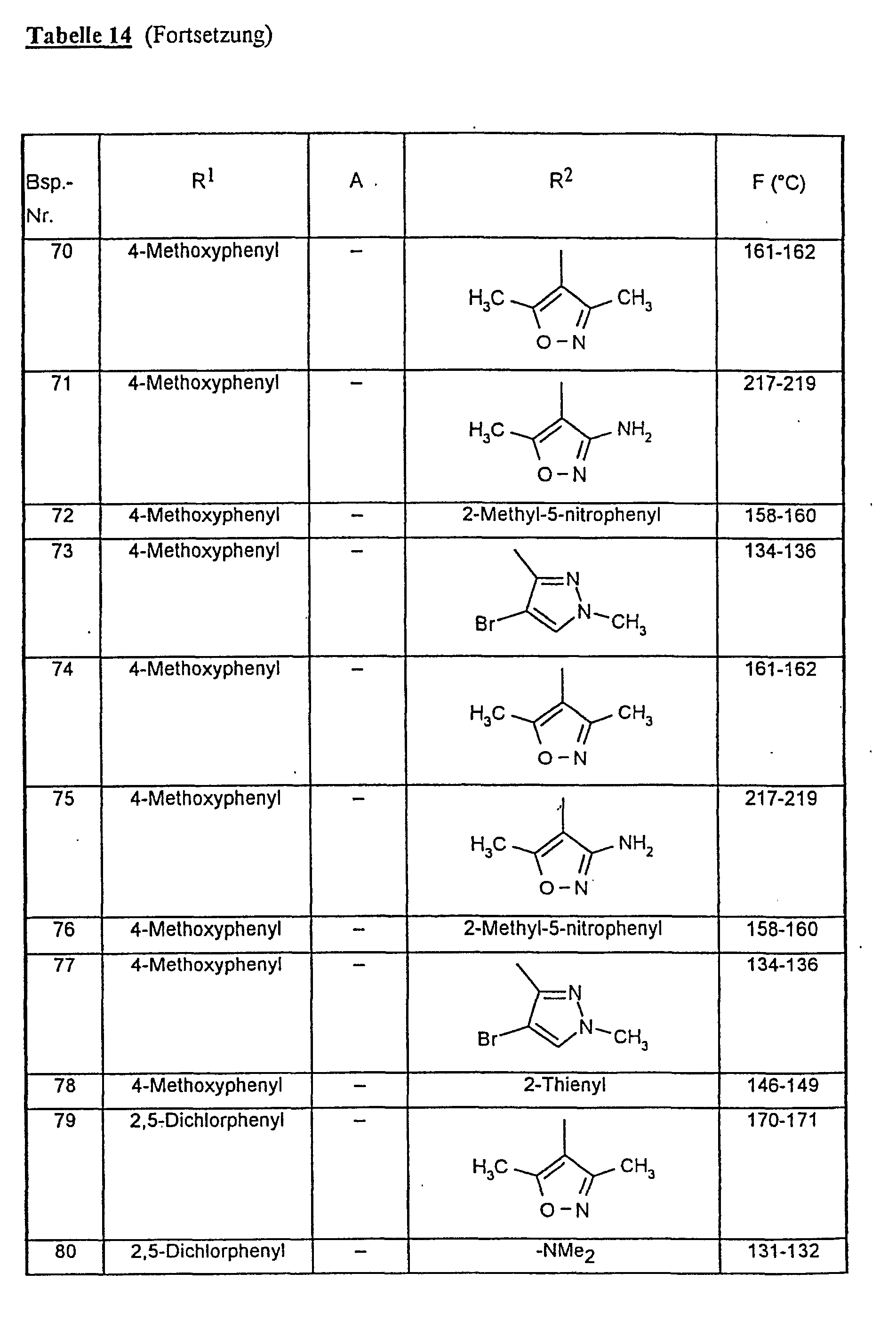

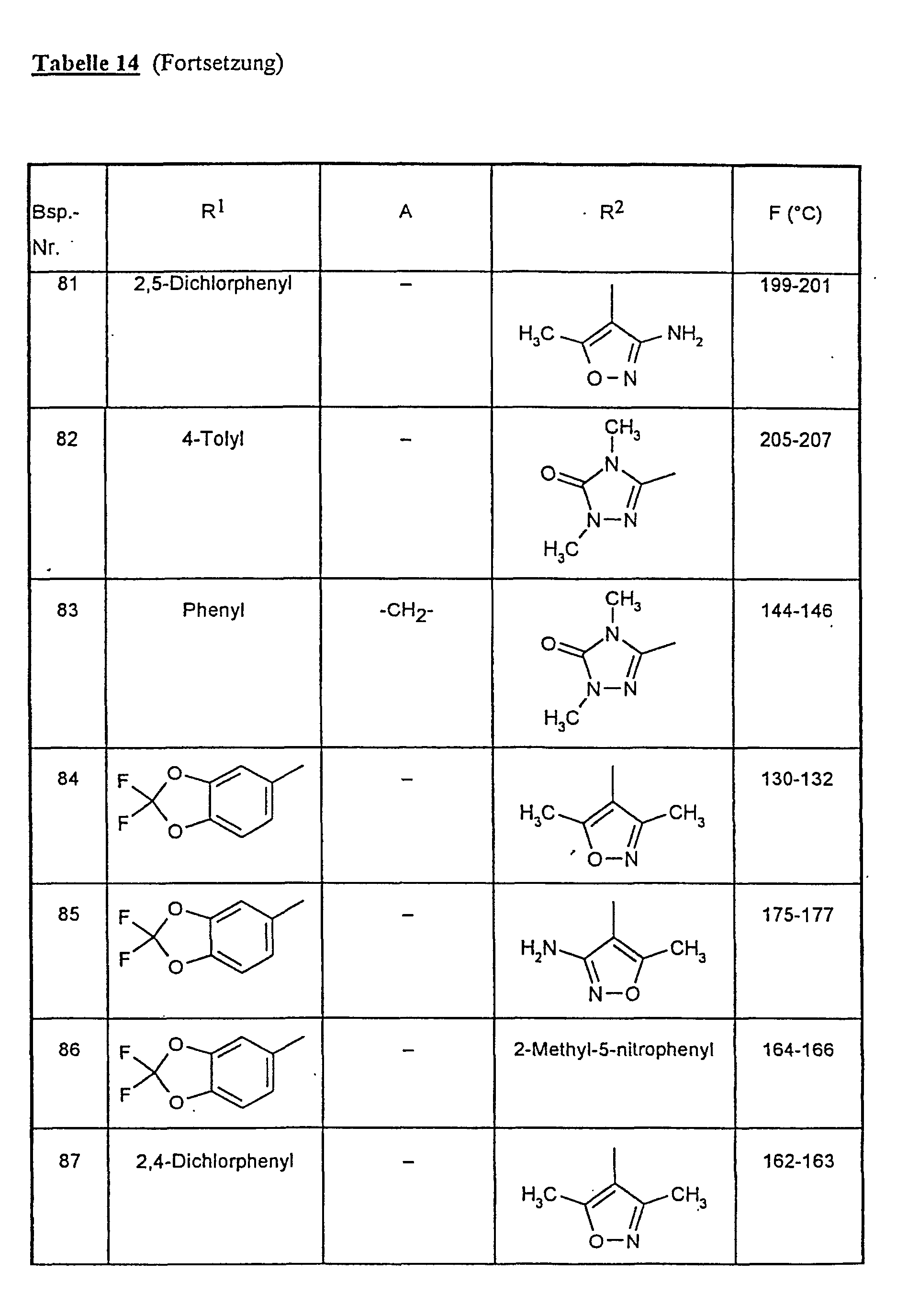

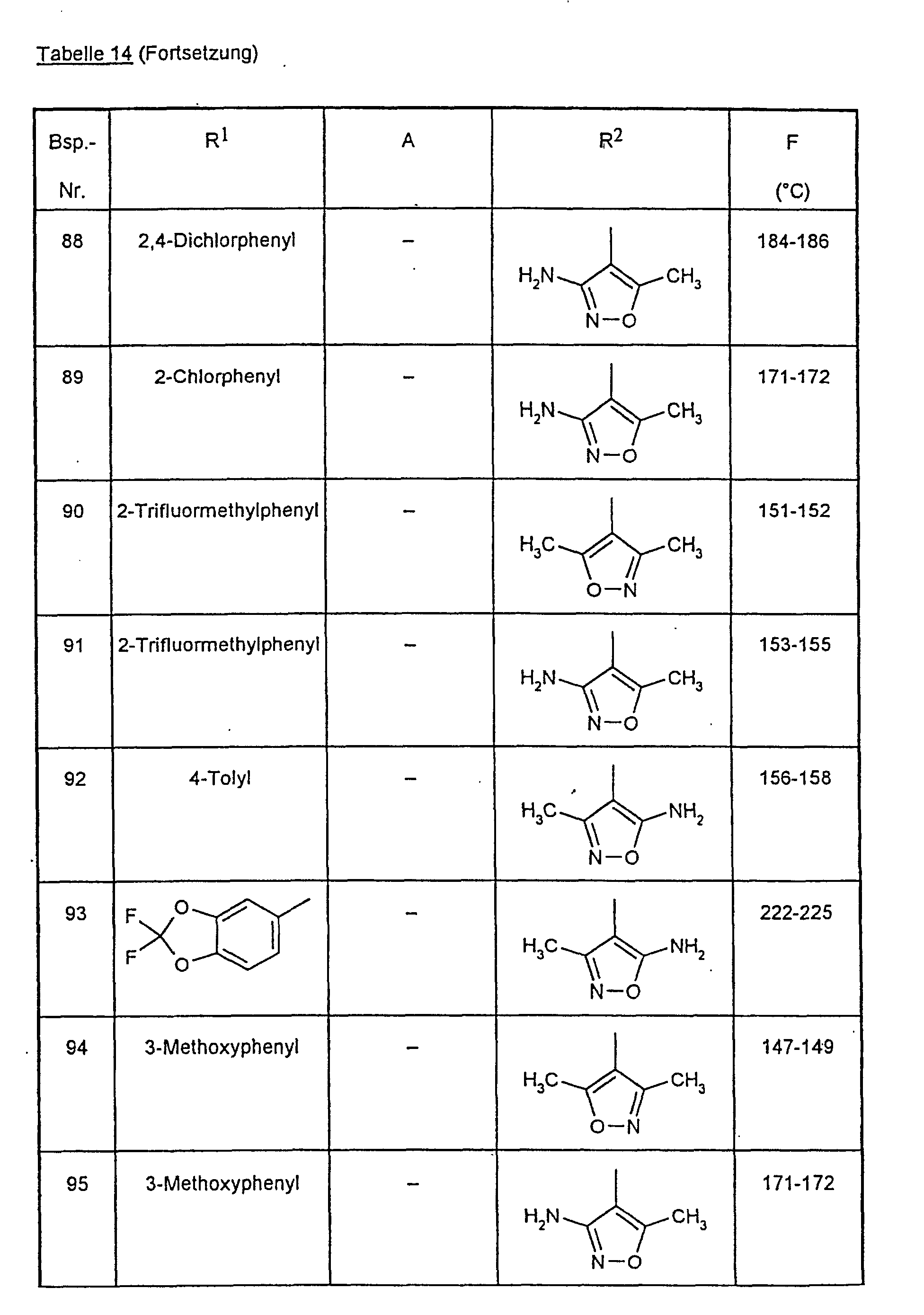

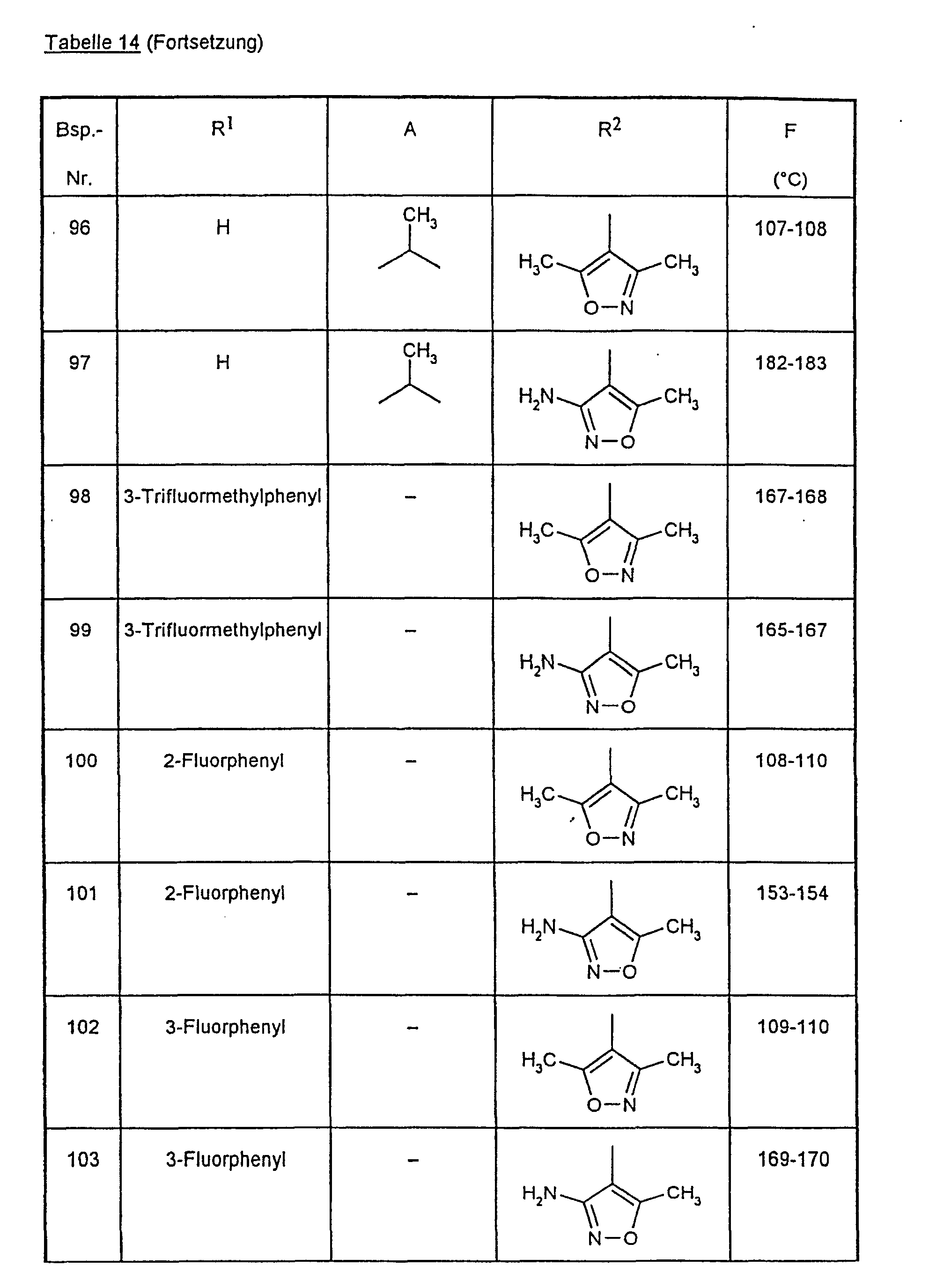

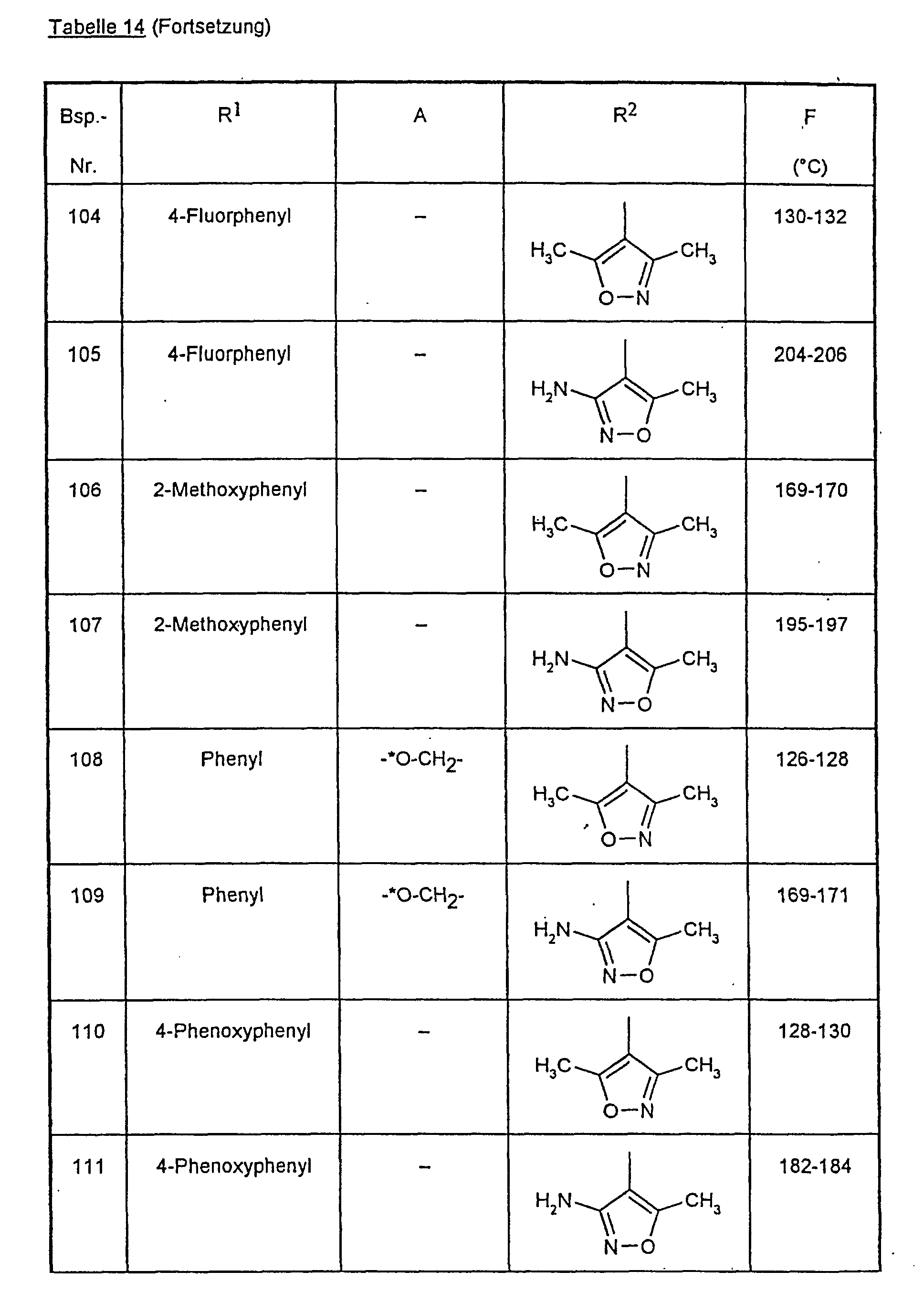

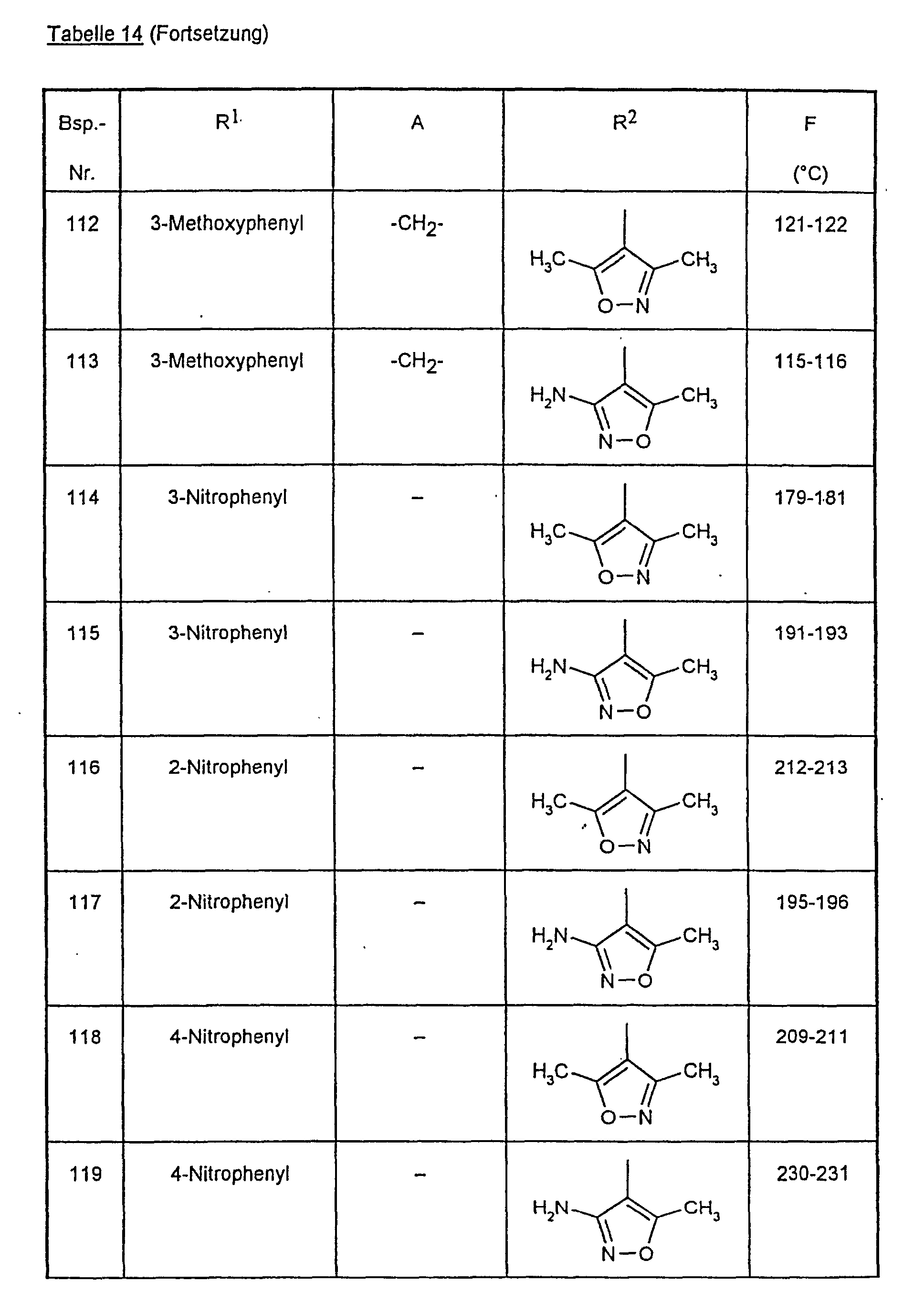

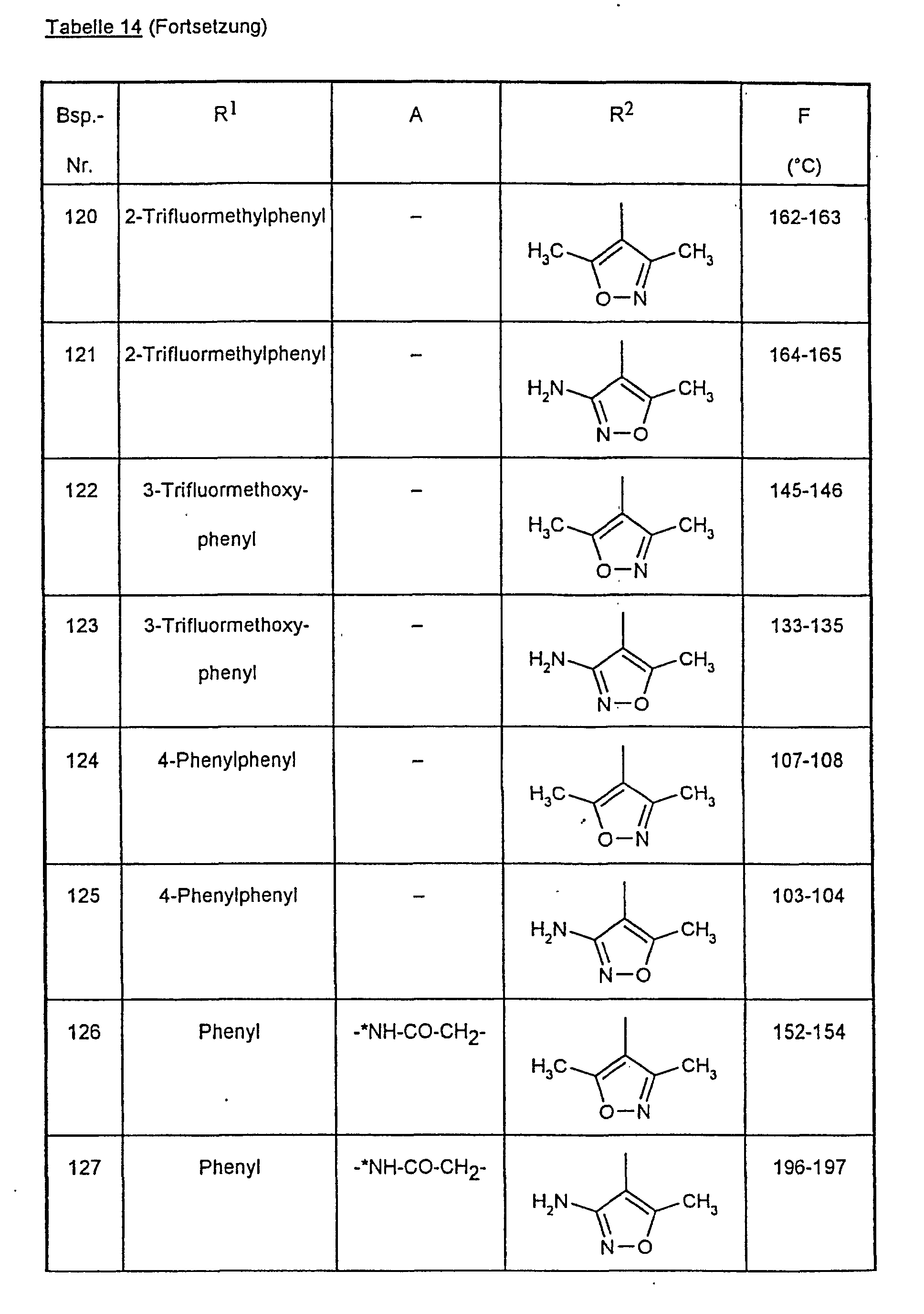

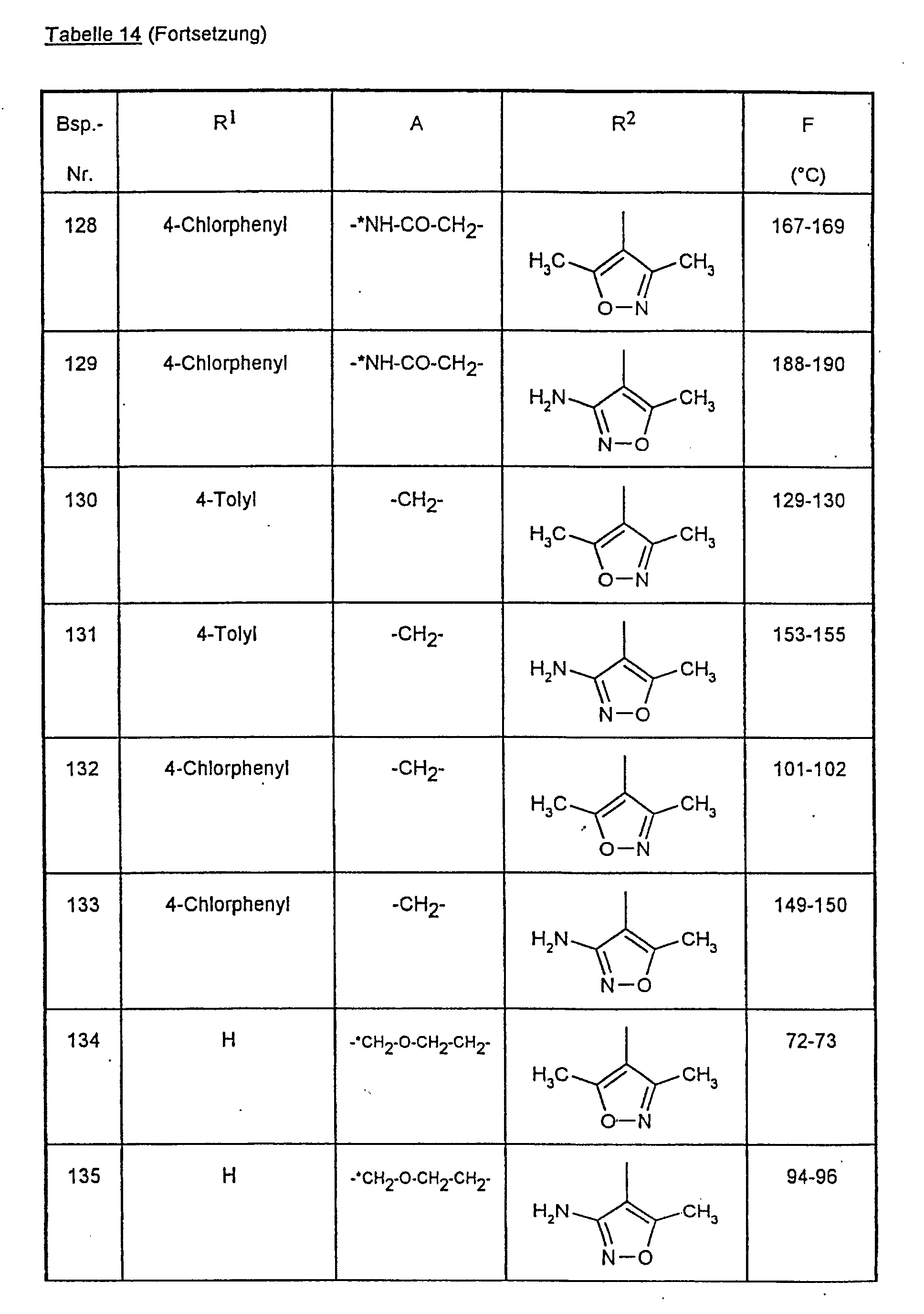

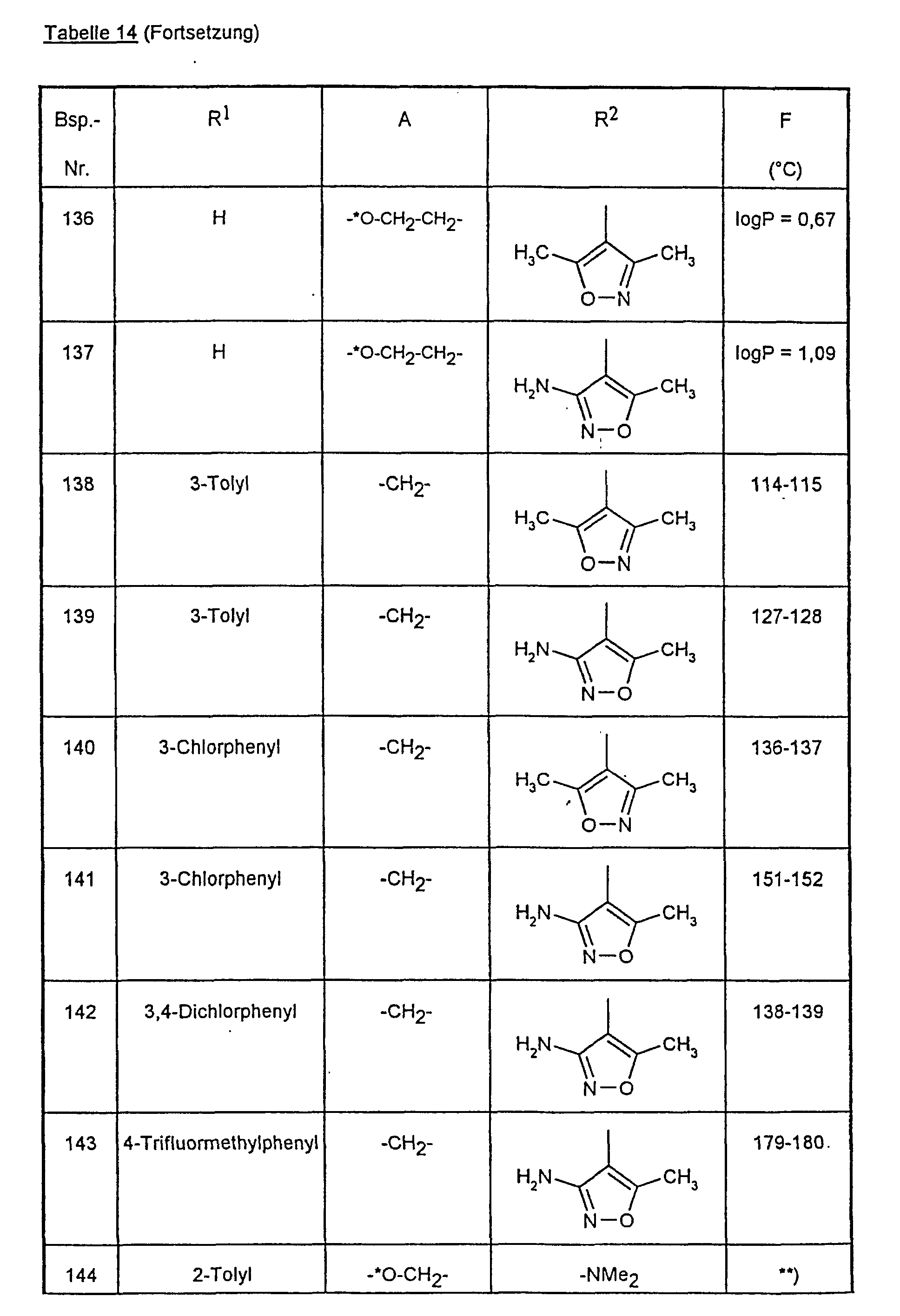

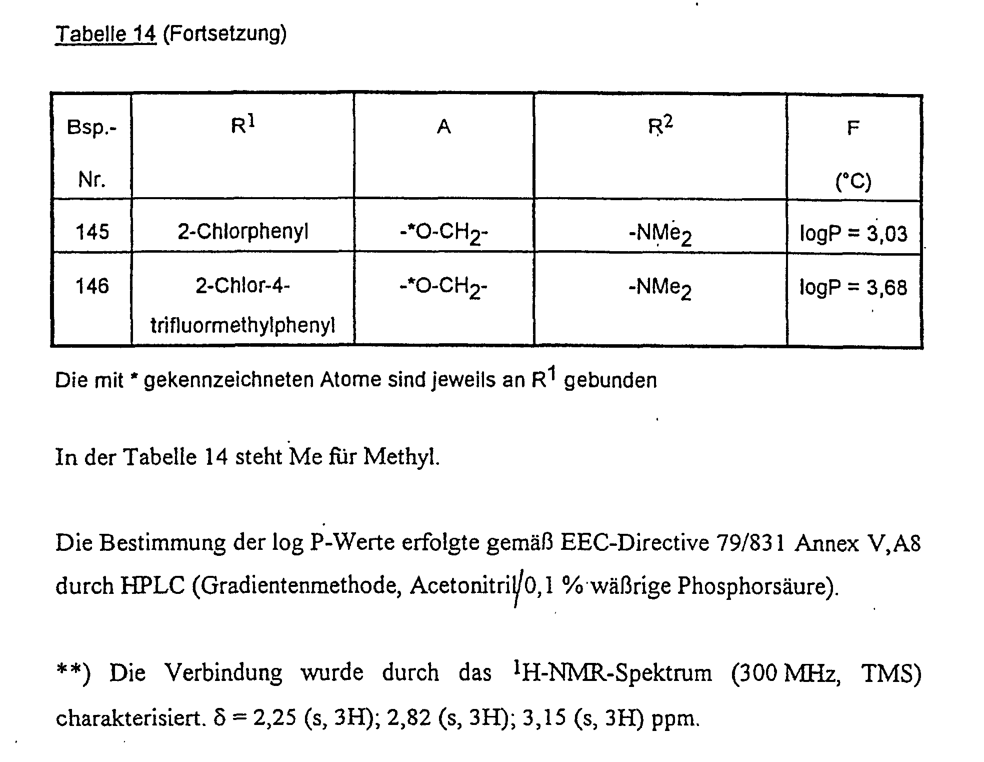

### Verwendungsbeispiele

### Beispiel A

Phytophthora-Test (Tomate) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

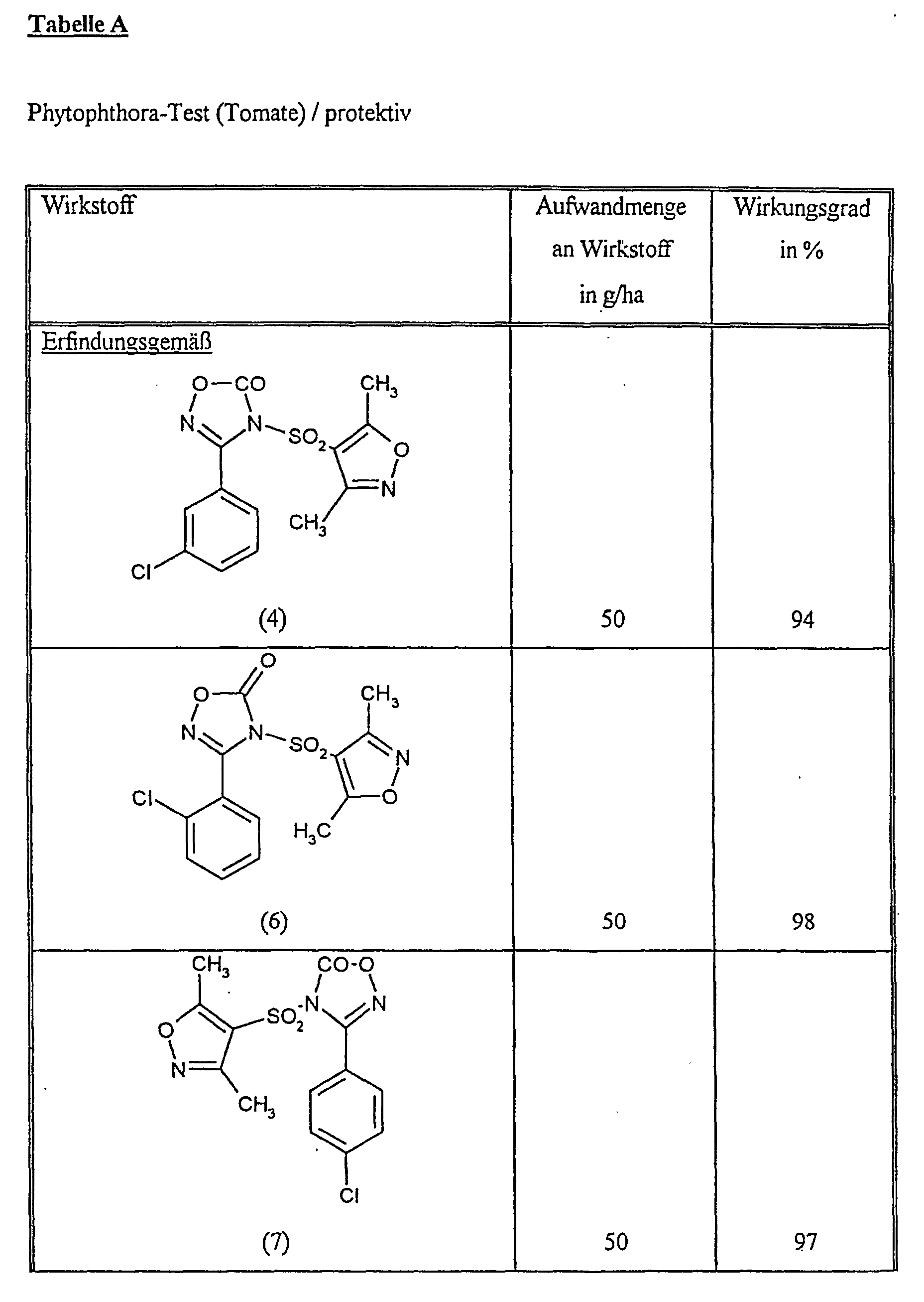

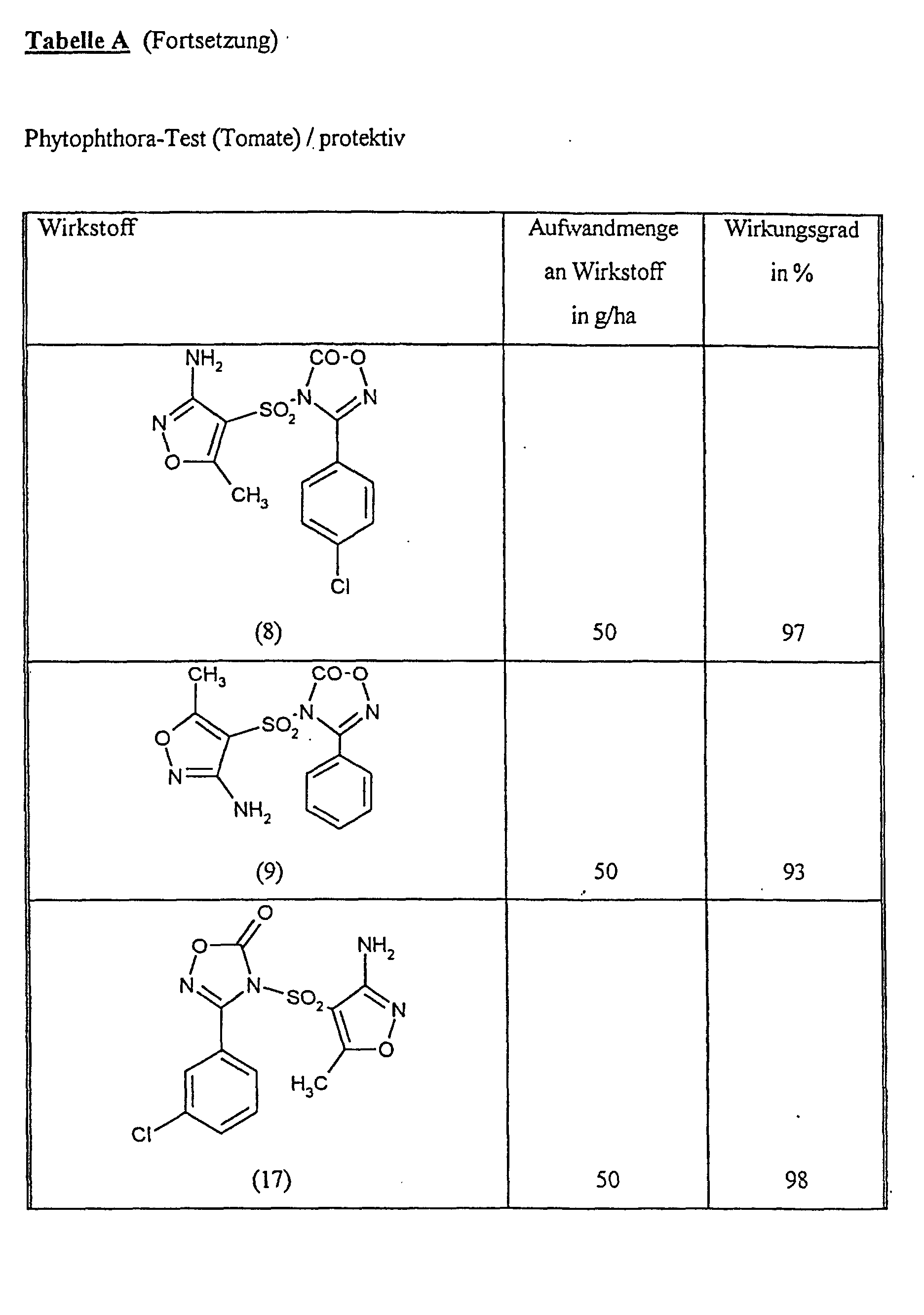

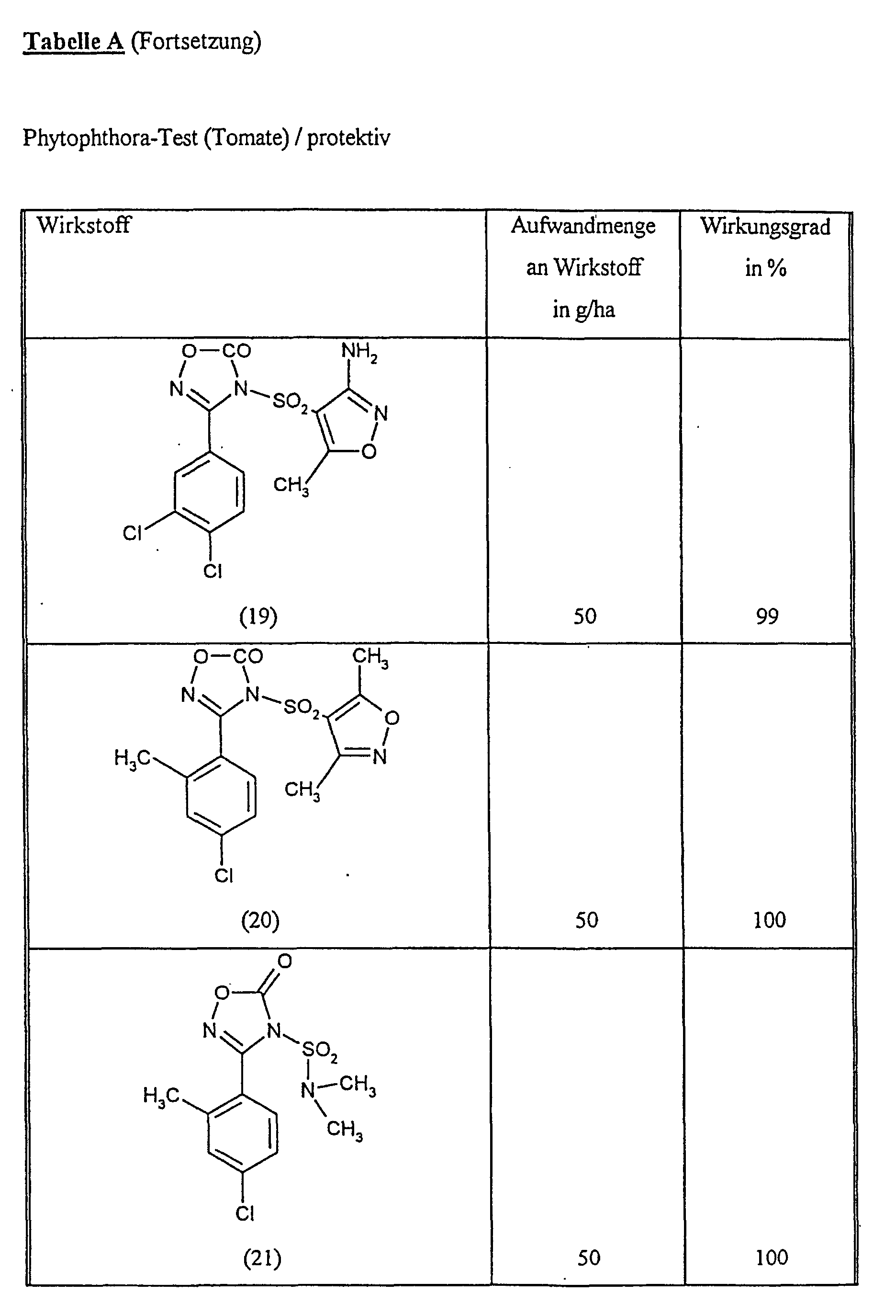

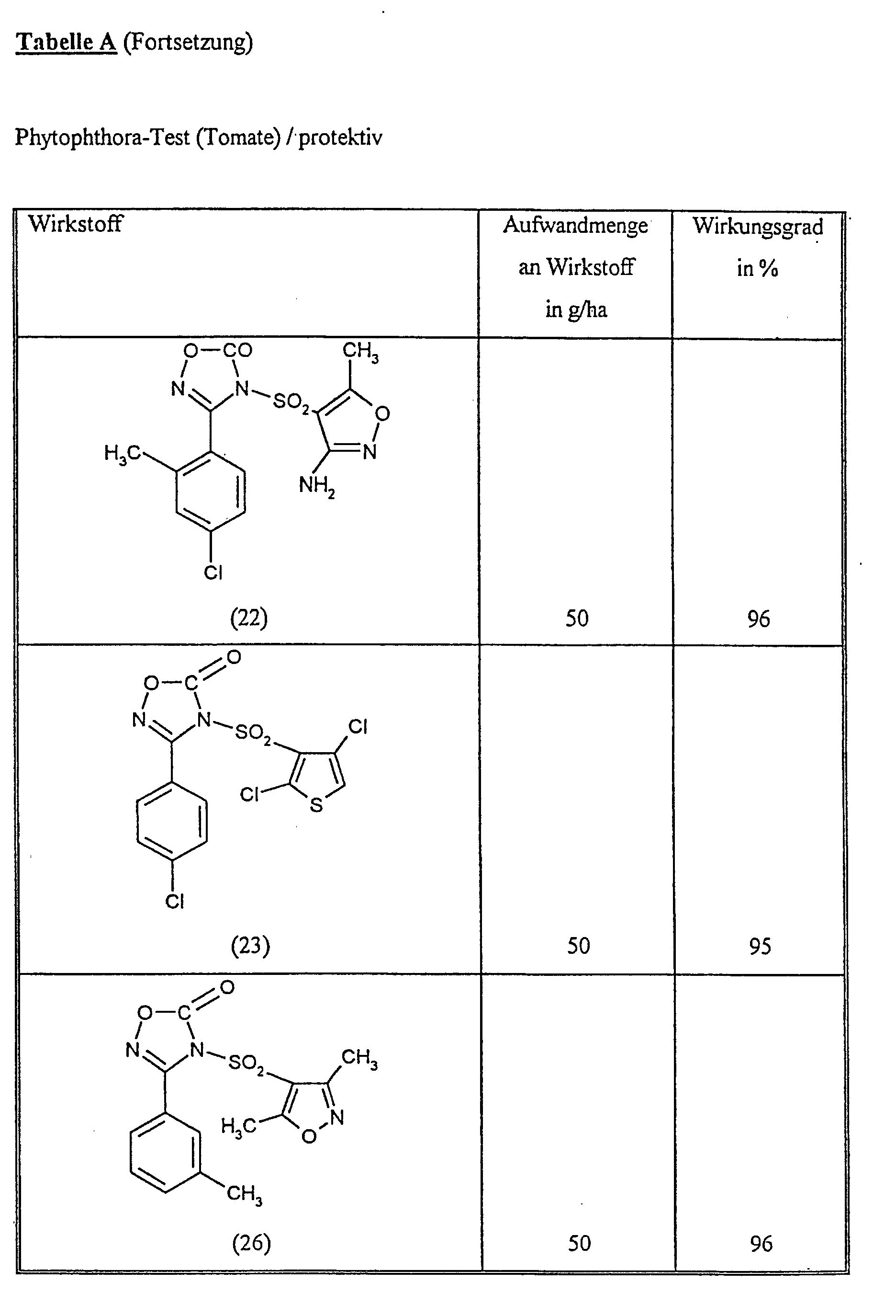

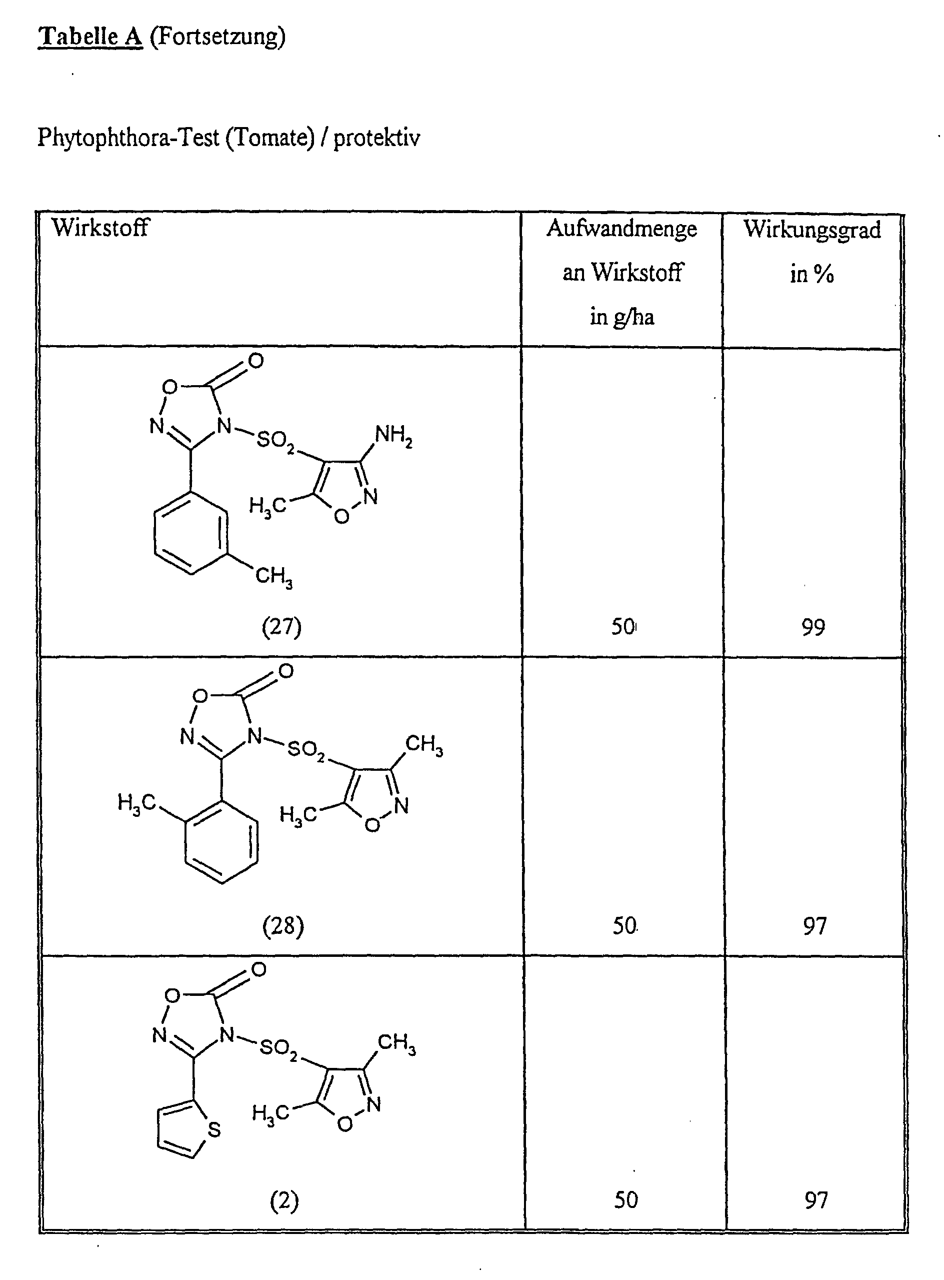

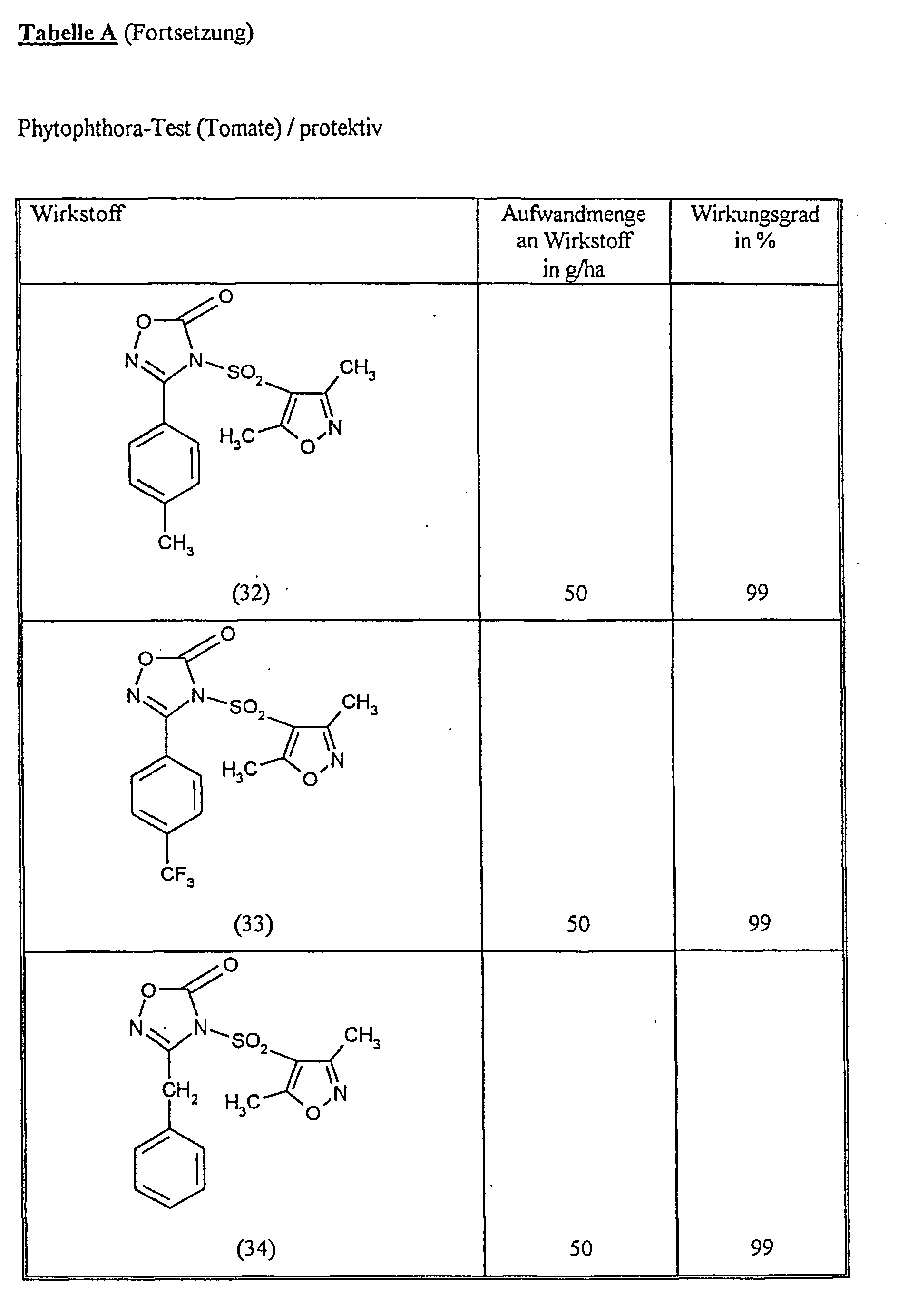

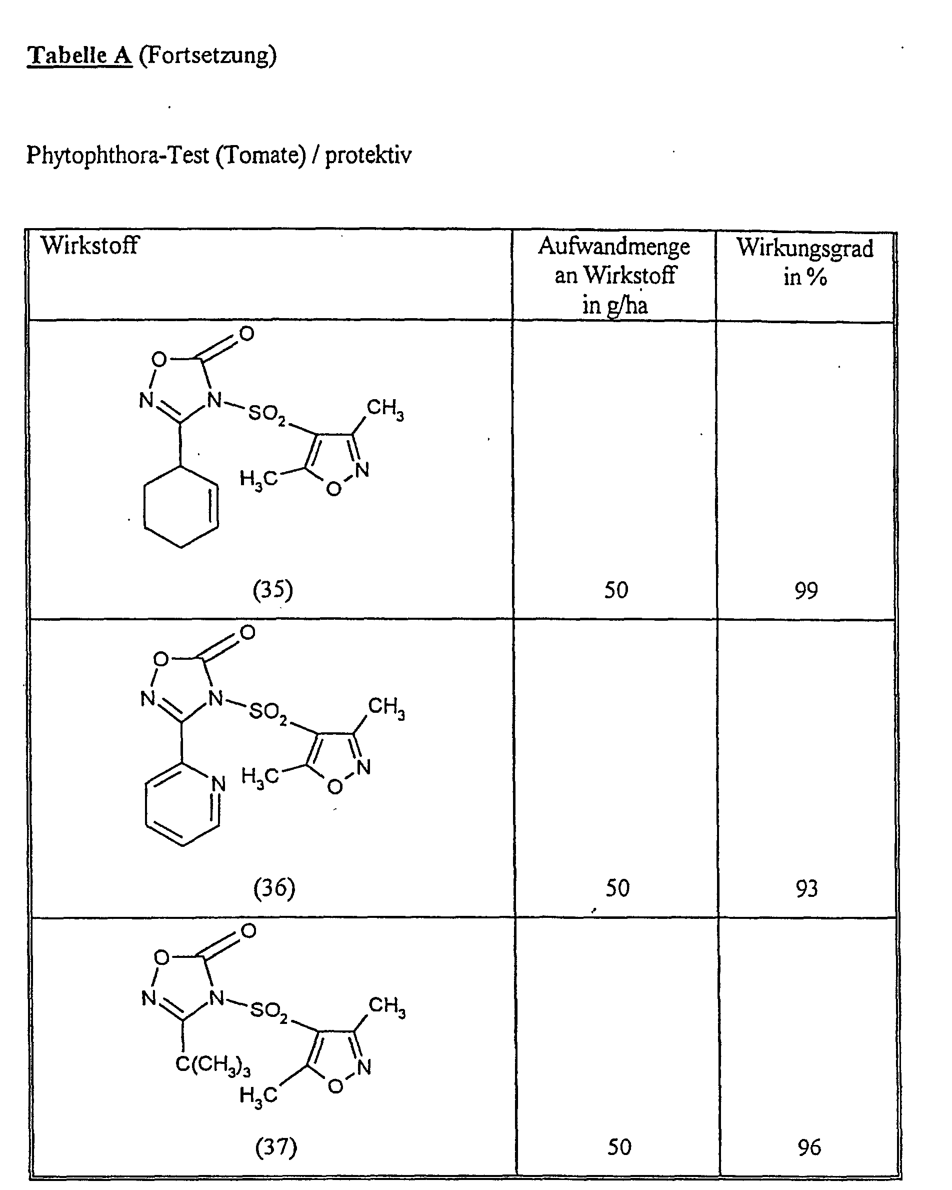

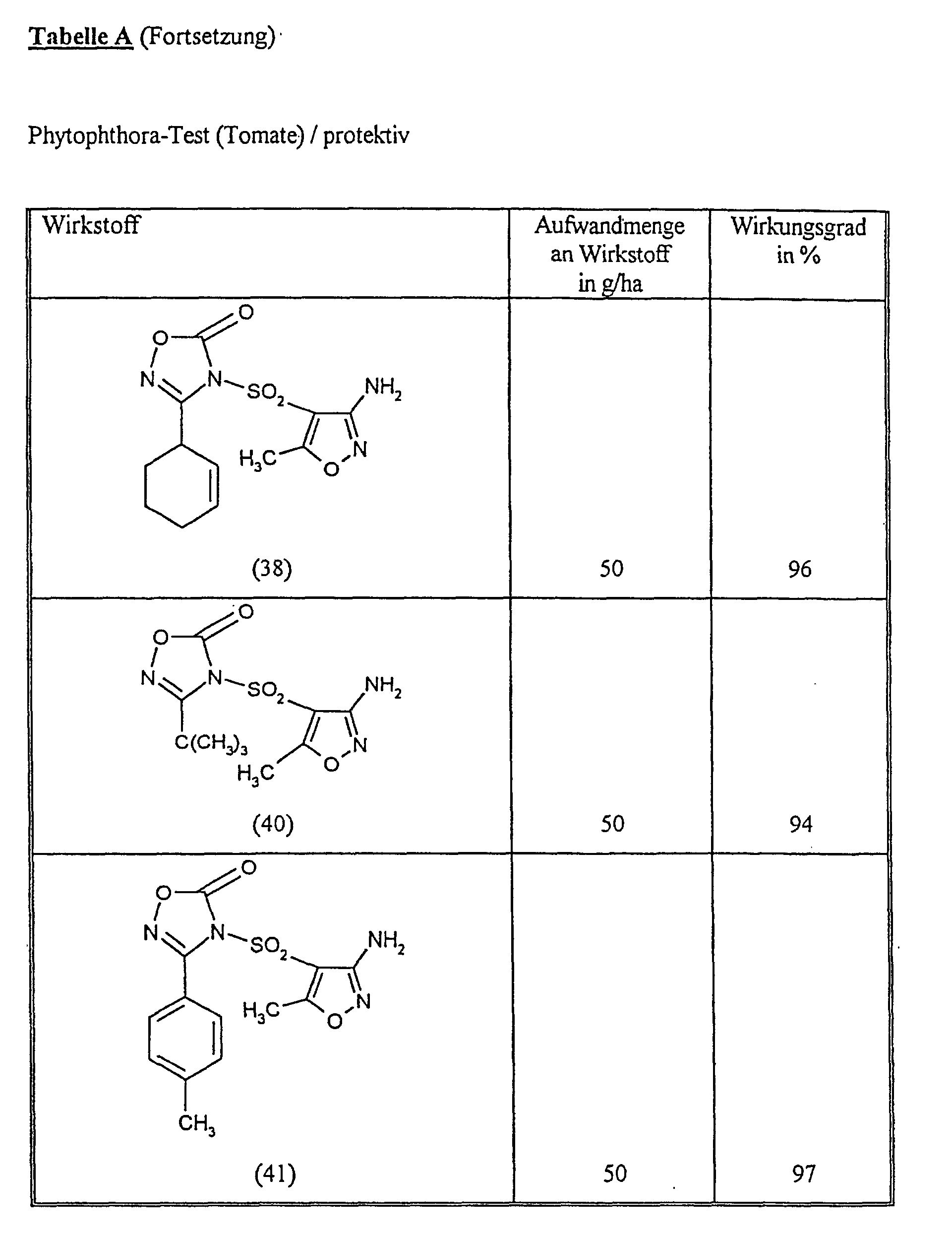

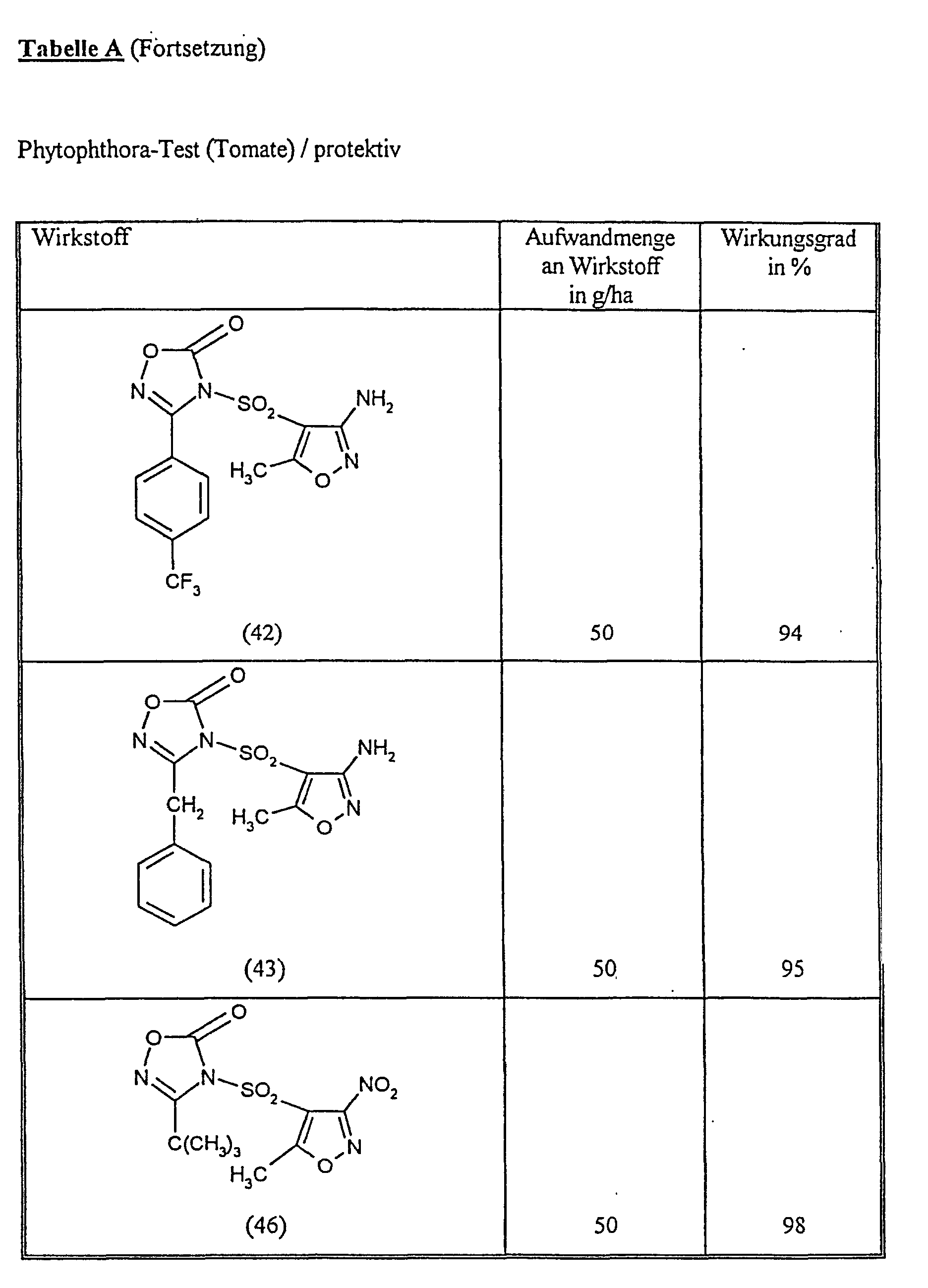

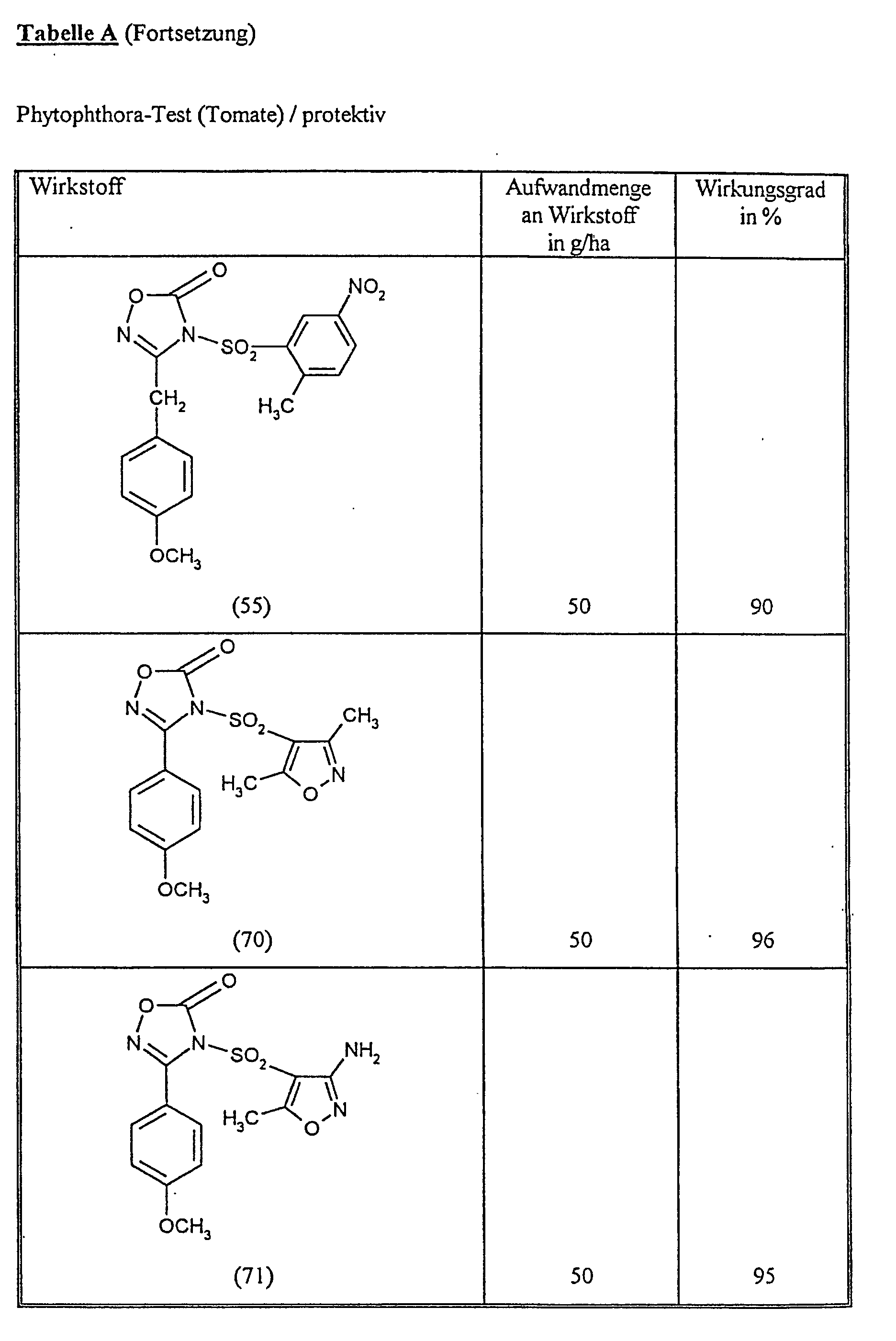

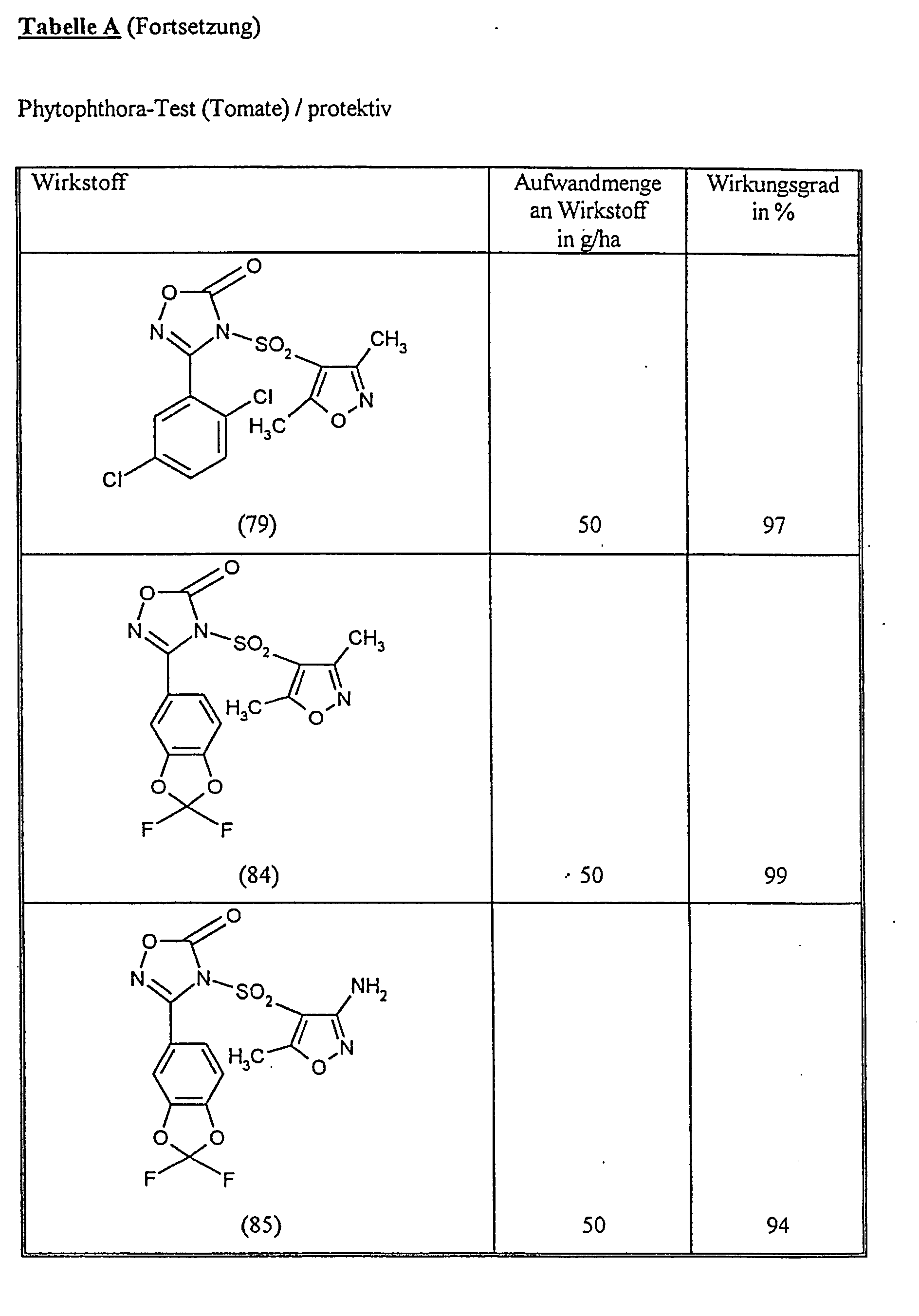

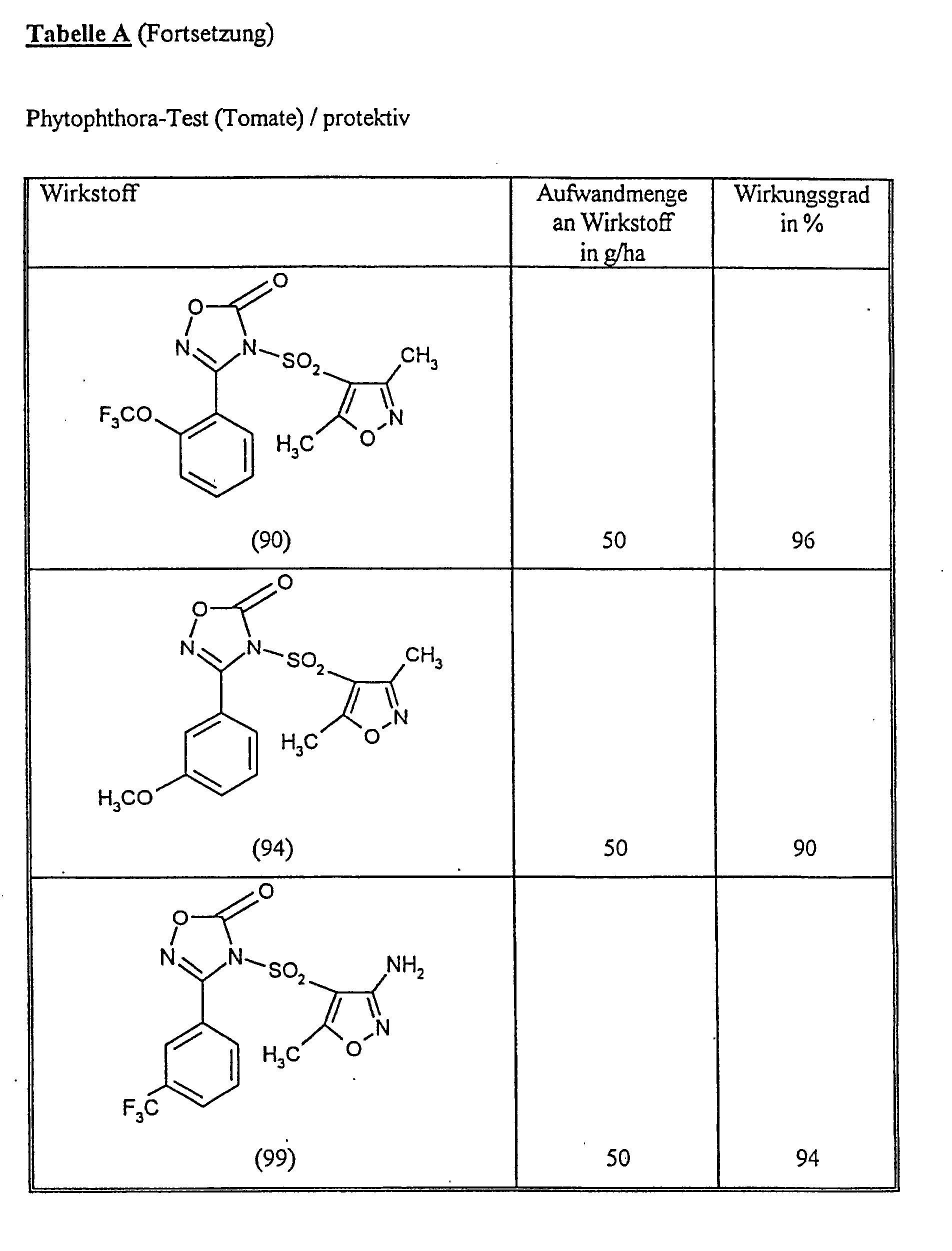

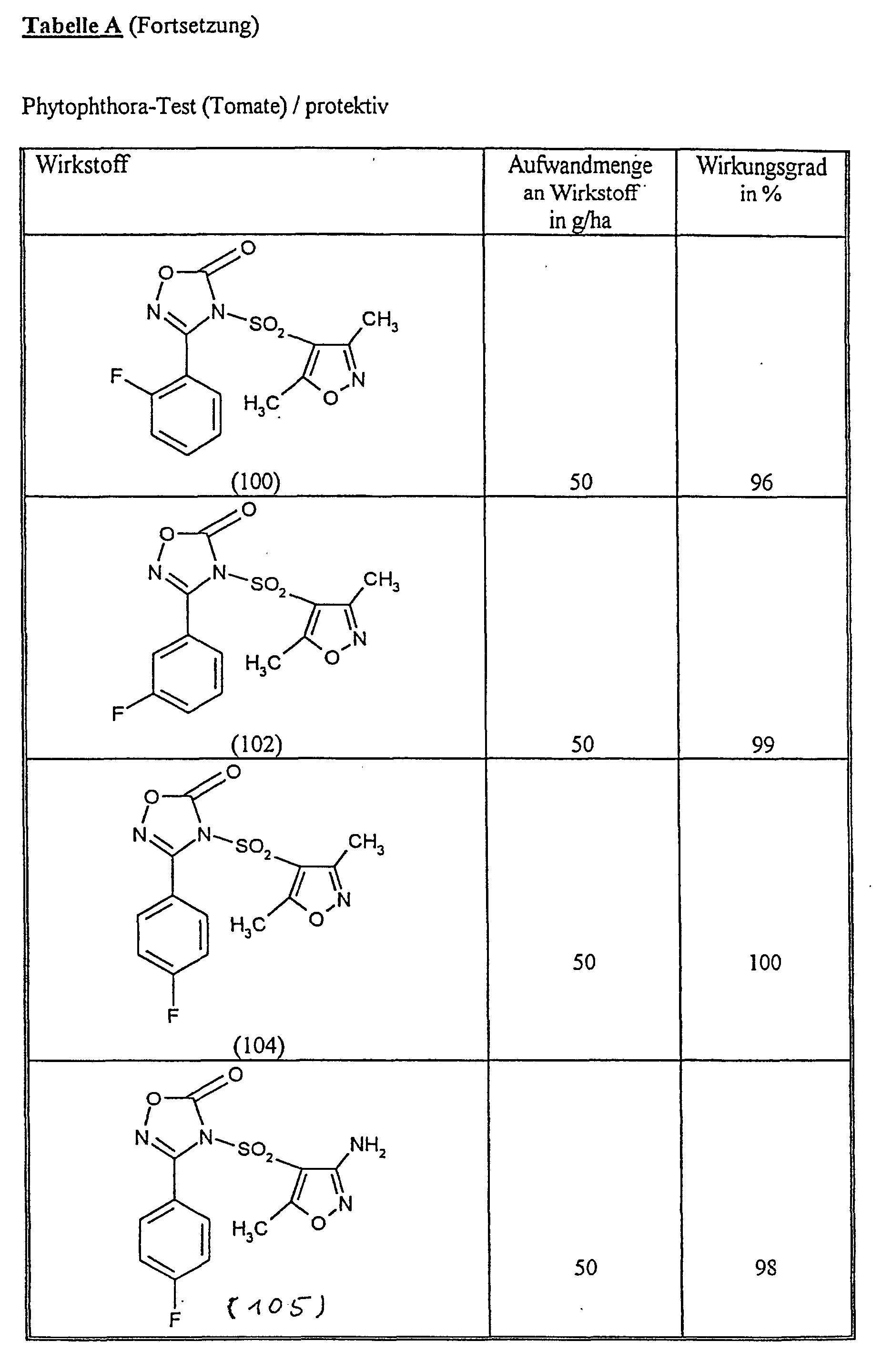

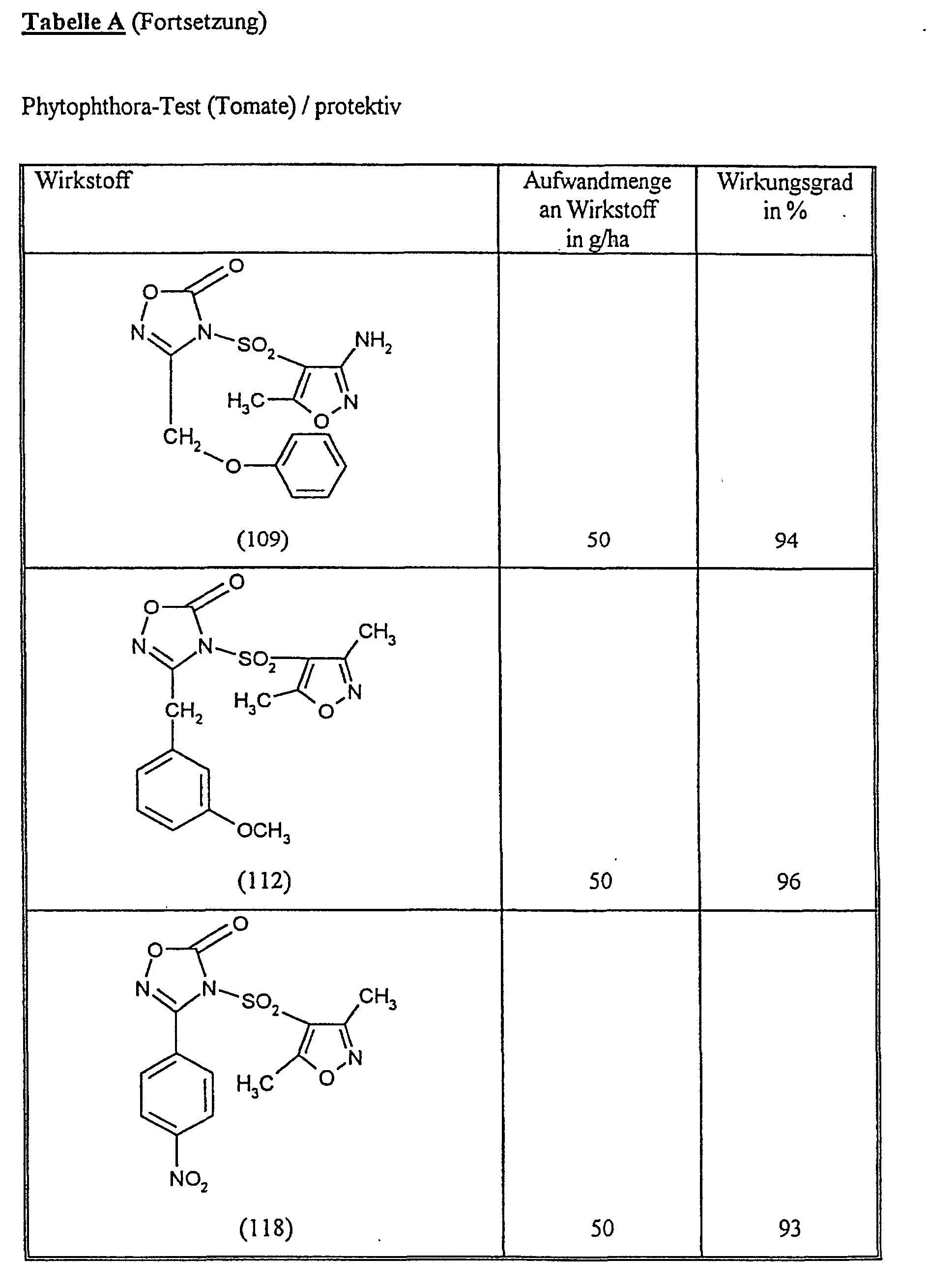

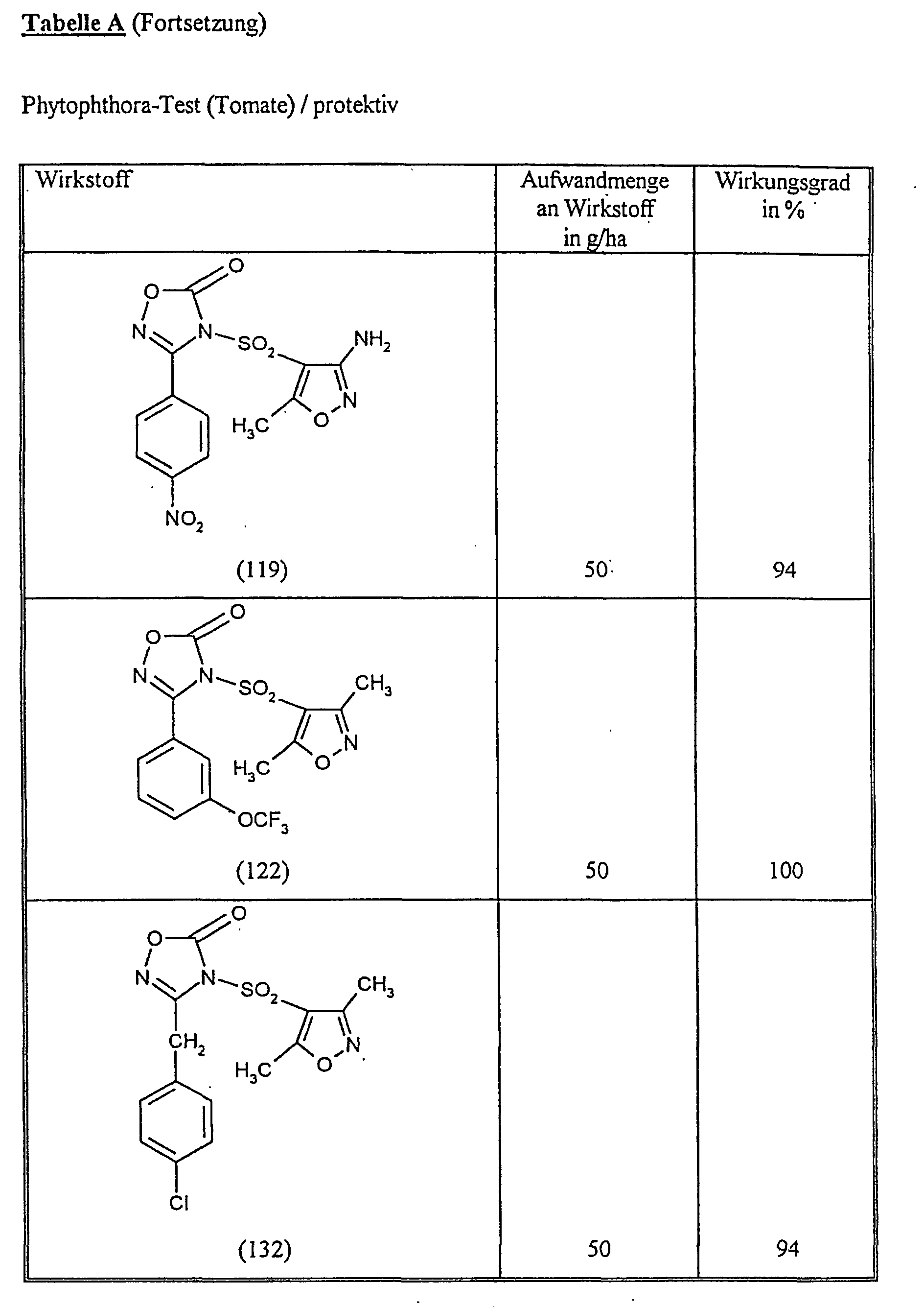

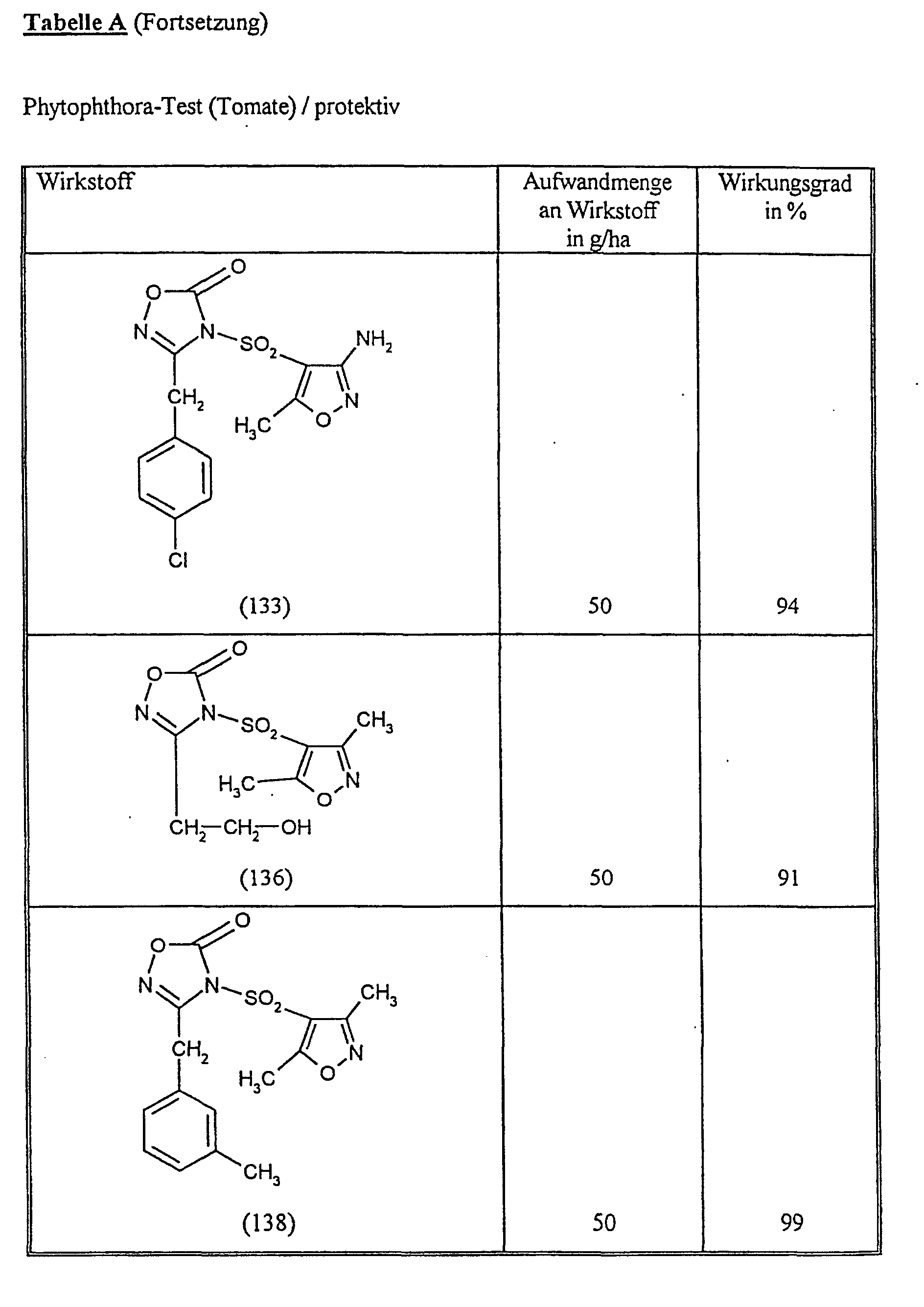

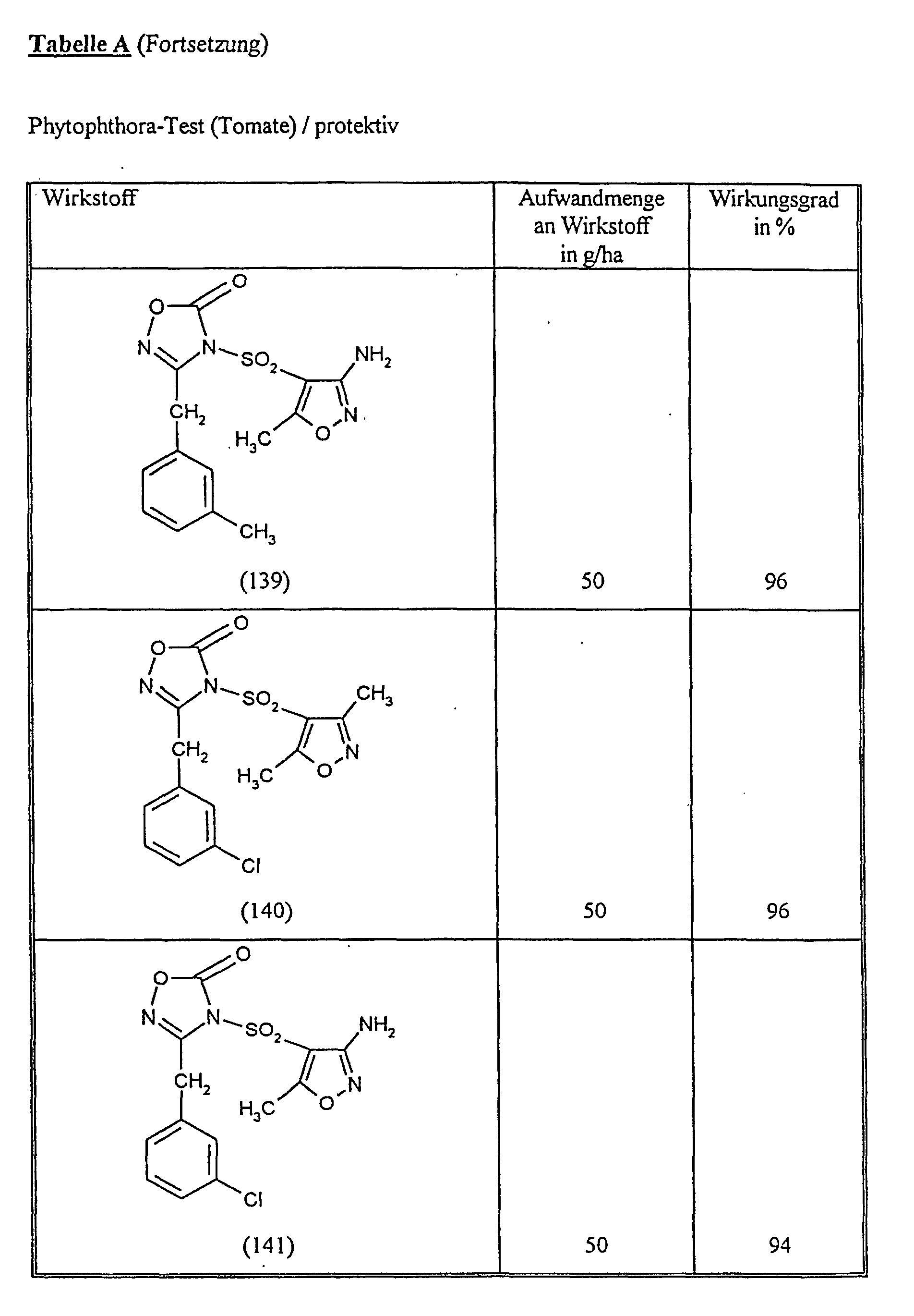

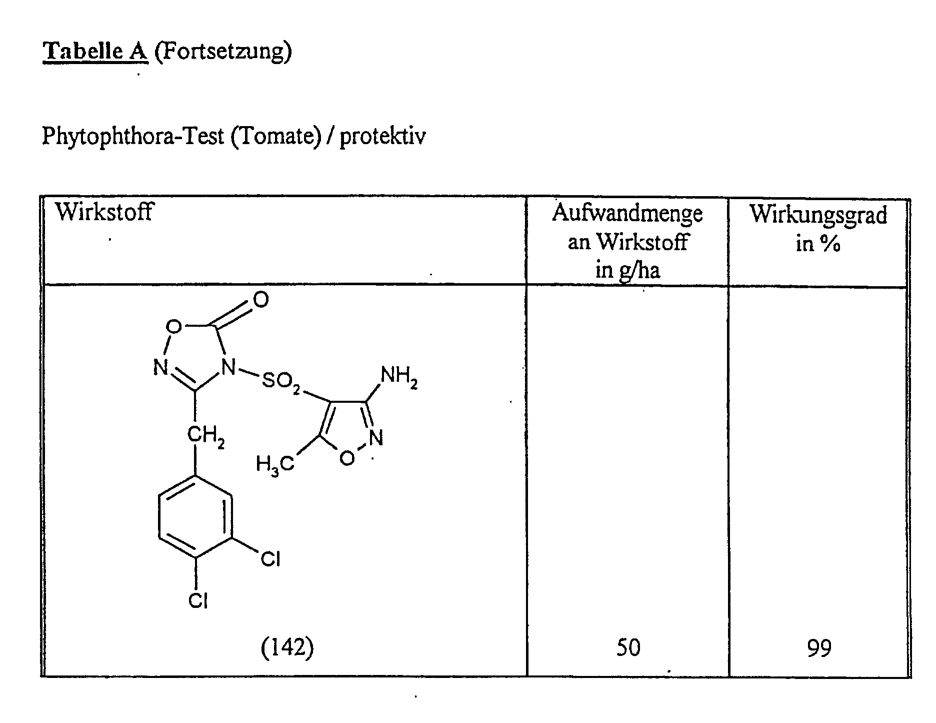

### Beispiel B

Plasmopara-Test (Rebe) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

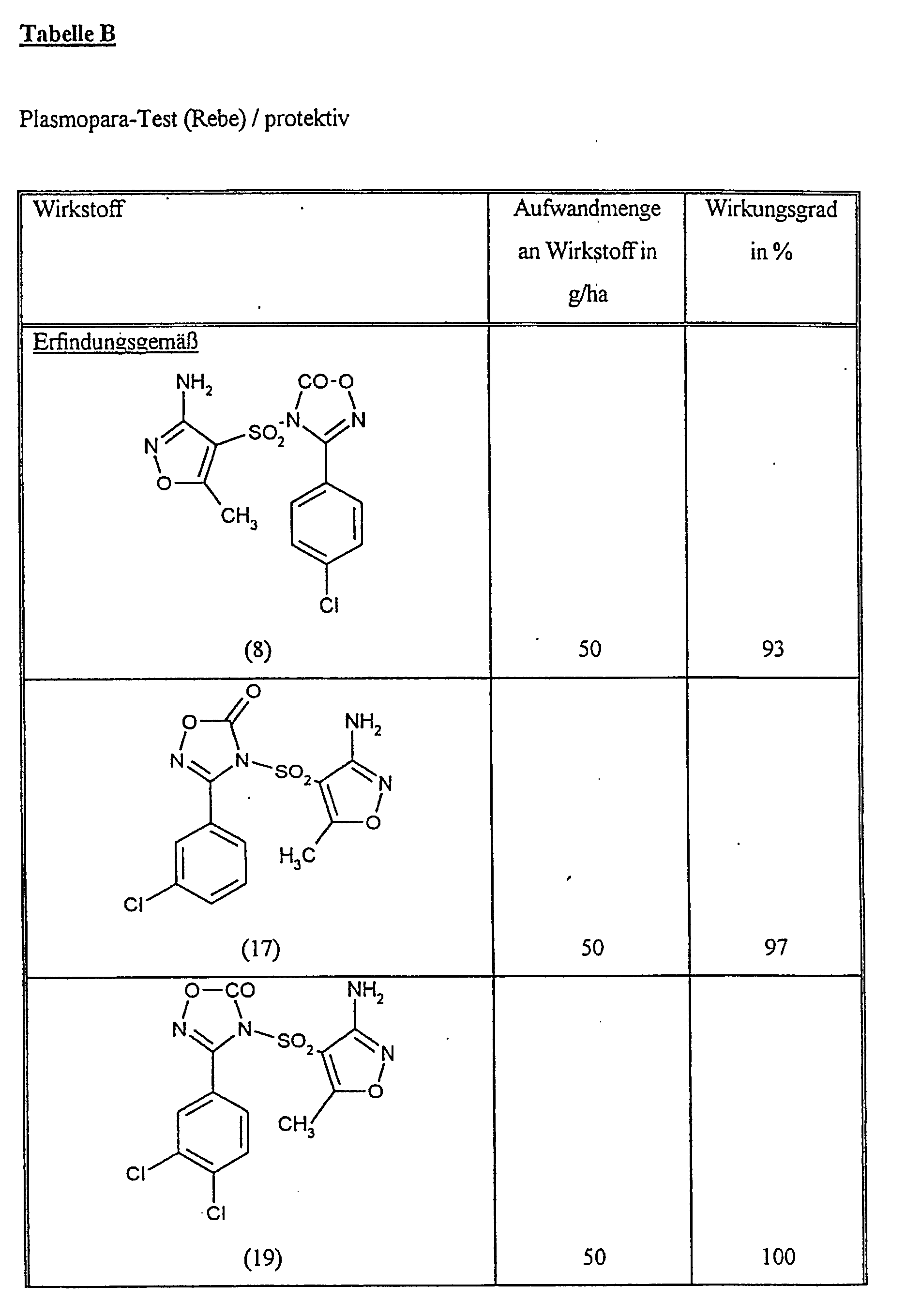

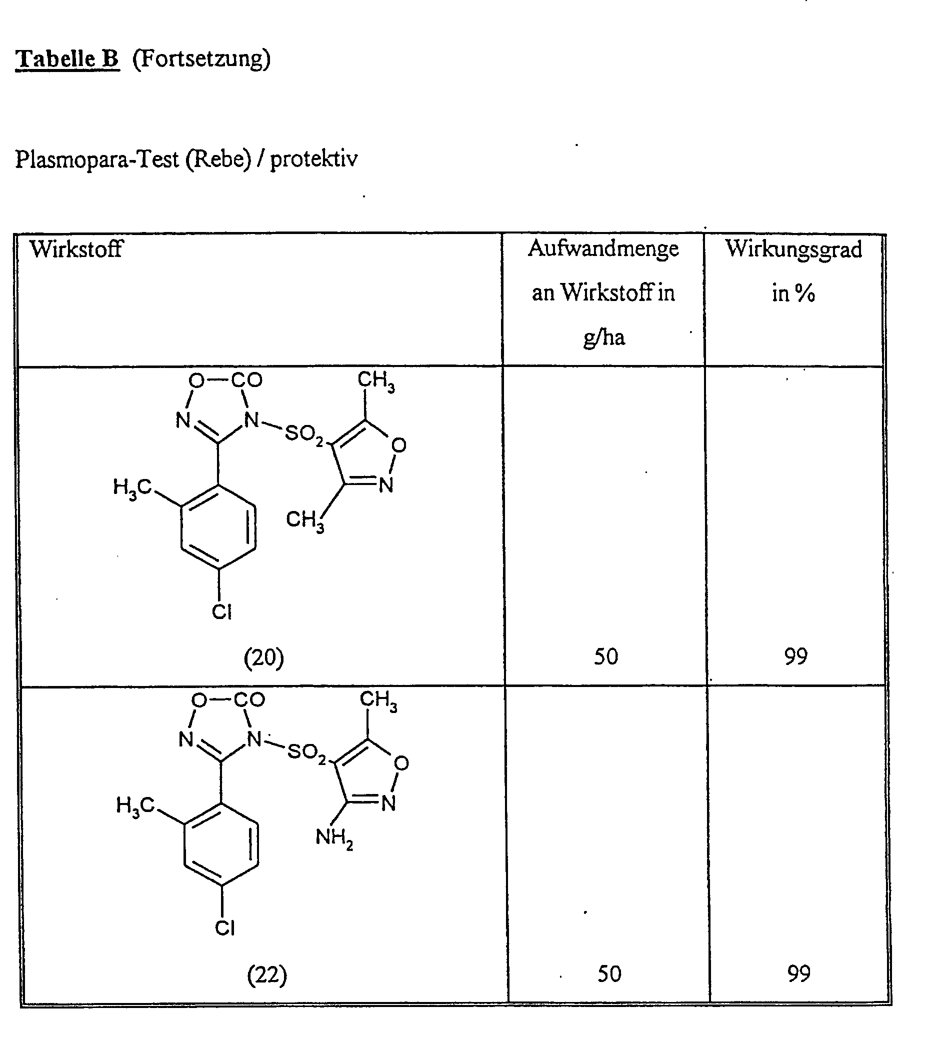

## Patentansprüche

1. Verwendung von Sulfonyloxadiazolonen der Formel 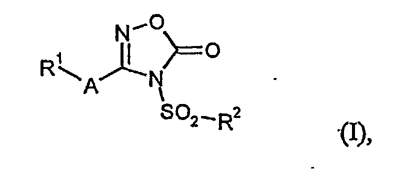 in welcher
A für eine Einfachbindung oder für Methandiyl, 1,1-Ethandiyl, 1,2-Ethandiyl 1,1-, 1,2-, 1,3- oder 2,2-Propandiyl, 1,1-, 1,2-, 1,3-, 1,4-, 2,2-, 2,3-Butandiyl, 1,1-, 1,2- oder 1,3-(2-Methyl-propandiyl), 1,1-Ethendiyl, 1,2-Ethendiyl 1,1-, 1,2- oder 1,3-Propendiyl, Ethindiyl, 1,3-Propindiyl oder eine Gruppierung -*O-CH₂-, -*O-CH₂-CH₂-, -*CH₂-O-, -*CH₂-O-CH₂-CH₂-, -*CH₂-S-, -*S-CH₂-, 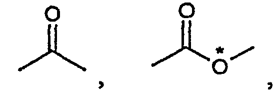 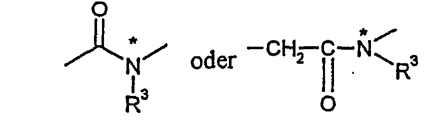 steht, wobei das mit * gekennzeichnete Atom jeweils mit R¹ verbunden ist,
R³ für Wasserstoff oder Methyl steht,
R¹ für Wasserstoff, oder für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl steht,
oder
R¹ für Phenyl steht das einfach bis dreifach, gleichartig oder verschieden substituiert sein kann durch Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl,
gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch Fluor, Chlor, Brom, Nitro, Methyl, Ethyl, n- oder i-Propyl, n-, i-, s- oder t-Butyl, Methoxy, Ethoxy, n-oder i-Propoxy, Methylthio, Ethylthio, n- oder i-Propylthio, Trifluormethyl, Trifluorethyl, Difluormethoxy, Trifluormethoxy, Difluorchlormethoxy oder Trifluorethoxy,
oder
R¹ für Thienyl, Furyl oder Pyridyl steht, wobei diese Reste einfach bis dreifach, gleichartig oder verschieden substituiert sein können durch Fluor, Chlor, Brom, Nitro, Amino, Hydroxy, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl und/oder Cyclohexyl,
und/oder durch
Phenyl, Phenoxy, Phenylthio, Benzyl, Benzyloxy und/oder Benzylthio, wobei diese aromatischen Reste einfach bis dreifach, gleichartig oder verschieden substituiert sein können durch Fluor, Chlor, Brom, Nitro, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy, Trifluorethoxy, Difluormethylthio, Difluorchlormethylthio und/oder Trifluormethylthio,
und
R² für Methyl, Ethyl, n- oder i-Propyl, n-, i-, s-, oder t-Butyl, Dimethylamino oder Diethylamino steht,
oder
R² für Phenyl steht, das einfach bis dreifach, gleichartig oder verschieden substituiert sein kann durch Fluor, Chlor, Brom, Nitro, Methyl, Ethyl, Methoxy, Ethoxy, Methylthio, Ethylthio, Trifluormethyl, Trifluorethyl, Difluormethoxy, Trifluormethoxy, Difluorchlormethoxy, Trifluorethoxy, Difluormethylthio, Difluorchlormethylthio oder Trifluormethylthio,
oder
R² für Furyl, Thienyl, Oxazolyl, Isoxazolyl, Pyrazolyl, Imidazolyl, Thiazolyl, Isothiazolyl, Oxadiazolyl, Thiadiazolyl, Pyridinyl, Pyridazinyl, Pyrazinyl, 1,2,3-Triazinyl, 1,2,4-Triazinyl 1,3,5-Triazinyl, 1,2,4-Triazolonyl Pyrrolidinyl, Piperidinyl oder Morpholinyl steht, wobei diese Reste einfach, zweifach oder dreifach, gleichartig oder verschieden substituiert sein können durch Fluor, Chlor, Brom, Cyano, Nitro, Amino, Hydroxy, Carbamoyl, Methyl, Ethyl, n- oder i-Propyl, n-, i-, s-, oder t-Butyl, Cyclopropyl, Methoxy, Ethoxy, n- oder i-Propoxy, Trifluormethyl, Trifluorethyl, Difluormethoxy, Trifluormethoxy, Acetyl, Propionyl, Methoxycarbonyl, Ethoxycarbonyl, Hydroxyiminomethyl, Hydroxyiminoethyl, Methoxyiminomethyl, Ethoxyiminomethyl, Methoxyiminoethyl und/oder Ethoxyiminoethyl,
zur Bekämpfung von unerwünschten Mikroorganismen im Pflanzenschutz und im Materialschutz.

2. Verfahren zur Bekämpfung von unerwünschten Mikroorganismen im Pflanzenschutz und im Materialschutz, **dadurch gekennzeichnet, daß** man Sulfonyloxadiazolone der Formel (I) gemäß Anspruch 1 auf die Mikroorganismen und/oder deren Lebensraum ausbringt.

3. Sulfonyloxadiazolone der Formel 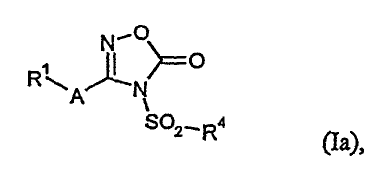 in welcher
A für eine Einfachbindung oder für Methandiyl, 1,1-Ethandiyl, 1,2-Ethandiyl 1,1-, 1,2-, 1,3- oder 2,2-Propandiyl, 1,1-, 1,2-, 1,3-, 1,4-, 2,2-, 2,3-Butandiyl, 1,1-, 1,2- oder 1,3-(2-Methyl-propandiyl), 1,1-Ethendiyl, 1,2-Ethendiyl 1,1-, 1,2- oder 1,3-Propendiyl, Ethindiyl, 1,3-, Propindiyl oder eine Gruppierung -*O-CH₂-, -*O-CH₂-CH₂-, -*CH₂-O-, -*CH₂-O-CH₂-CH₂-, -*CH₂-S-, -*S-CH₂-, 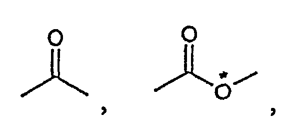 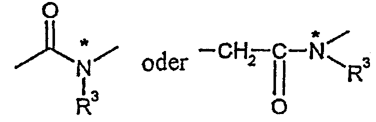 steht, wobei das mit * gekennzeichnete Atom jeweils mit R¹ verbunden ist,
R³ für Wasserstoff oder Methyl steht,
R¹ für Wasserstoff, oder für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl steht,
oder
R¹ für Phenyl steht, das einfach bis dreifach, gleichartig oder verschieden substituiert sein kann durch Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl,
gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobulyl, Cyclopentyl oder Cyclohexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch Fluor, Chlor, Brom, Nitro, Methyl, Ethyl, n- oder i-Propyl, n-, i-, s- oder t-Bulyl, Methoxy, Ethoxy, n-oder i-Propoxy, Methylthio, Ethylthio, n- oder i-Propylthio, Trifluormethyl, Trifluorethyl, Difluormethoxy, Trifluormethoxy, Difluorchlormethoxy oder Trifluorethoxy,
oder
R¹ für Thienyl, Furyl oder Pyridyl steht, wobei diese Reste einfach bis dreifach, gleichartig oder verschieden substituiert sein können durch Fluor, Chlor, Brom, Nitro, Amino, Hydroxy, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl und/oder Cyclohexyl,
und/oder durch
Phenyl, Phenoxy, Phenylthio, Benzyl, Benzyloxy und/oder Benzylthio, wobei diese aromatischen Reste einfach bis dreifach, gleichartig oder verschieden substituiert sein können durch Fluor, Chlor, Brom, Nitro, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy, Trifluorethoxy, Difluormethylthio, Difluorchlormethylthio und/oder Trifluormethylthio,
und
R⁴ für Dimethylamino oder Diethylamino steht,
oder
R⁴ für durch Nitro substituiertes Phenyl steht, das zusätzlich noch einfach oder zweifach, gleichartig oder verschieden substituiert sein kann durch Fluor, Chlor, Brom, Nitro, Methyl, Ethyl, Methoxy, Ethoxy, Methylthio, Ethylthio, Trifluormethyl, trifluorethyl, Difluormethoxy, Trifluormethoxy, Difluorchlormethoxy, Trifluorethoxy, Difluormethylthio, Difluorchlormethylthio oder Trifluormethylthio,
oder
R⁴ für Furyl, Thienyl, Oxazolyl, Isoxazolyl, Pyrazolyl, Imidazolyl, Thiazolyl, Isothiazolyl, Oxadiazolyl, Thiadiazolyl, Pyridinyl, Pyridazinyl, Pyrazinyl, 1,2,3-Triazinyl, 1,2,4-Triazinyl, 1,3,5-Triazinyl, 1,2,4-Triazolonyl, Pyrrolidinyl, Piperidinyl oder Morpholinyl, steht, wobei diese Reste einfach, zweifach oder dreifach, gleichartig oder verschieden substituiert sein können durch Fluor, Chlor, Brom, Cyano, Nitro, Amino, Hydroxy, Carbamoyl, Methyl, Ethyl, n- oder i-Propyl, n-, i-, s-, oder t-Butyl, Cyclopropyl, Methoxy, Ethoxy, n- oder i-Propoxy, Trifluormethyl, Trifluorethyl, Difluormethoxy, Trifluormethoxy, Acetyl, Propionyl, Methoxycarbonyl, Ethoxycarbonyl, Hydroxyiminomethyl, Hydroxyiminoethyl, Methoxyiminomethyl, Ethoxyiminomethyl, Methoxyiminoethyl und/oder Ethoxyiminoethyl.

4. Verfahren zur Herstellung von Sulfonyloxadiazolonen der Formel (Ia) gemäß Anspruch 3, **dadurch gekennzeichnet, daß** man
Oxadiazolone der Formel 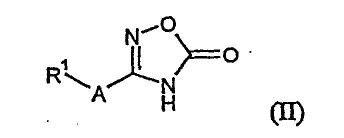 in welcher
A und R¹ die oben angegebenen Bedeutungen haben,
mit Sulfonsäurehalogeniden der Formel
R⁴-SO₂-X (III)
in welcher
R⁴ die oben angegebene Bedeutung hat und
X für Chlor steht,
gegebenenfalls in Gegenwart eines Säurebindemittels und gegebenenfalls in Gegenwart eines Verdünnungsmittels umsetzt.

5. Mikrobizide Mittel, **gekennzeichnet durch** einen Gehalt an mindestens einem Sulfonyloxadiazolon der Formel (Ia) gemäß Anspruch 3 neben Streckmitteln und/oder oberflächenaktiven Stoffen.

6. Verfahren zur Herstellung von mikrobiziden Mitteln, **dadurch gekennzeichnet, daß** man Sulfonyloxadiazolone der Formel (Ia) gemäß Anspruch 3 mit Streckmitteln und/oder oberflächenaktiven Stoffen vermischt.

7. Sulfonyloxadiazolon gemäß Anspruch 3, **gekennzeichnet durch** die Formel

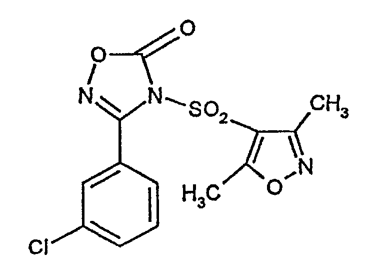

8. Sulfonyloxadiazolon gemäß Anspruch 3, **gekennzeichnet durch** die Formel

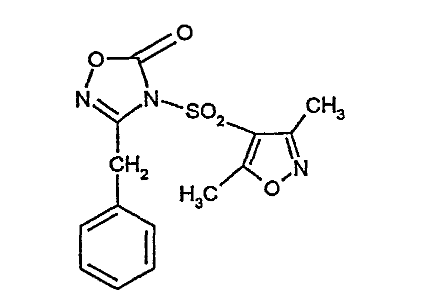

9. Sulfonyloxadiazolon gemäß Anspruch 3, **gekennzeichnet durch** die Formel

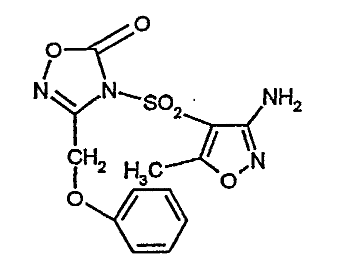

## Claims

1. Use of sulphonyloxadiazolones of the formula 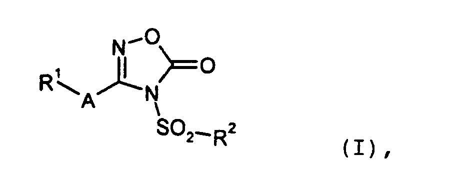 in which
A represents a single bond or represents methanediyl, 1,1-ethanediyl, 1,2-ethanediyl, 1,1-, 1,2-, 1,3- or 2,2-propanediyl, 1,1-, 1,2-, 1,3-, 1,4-, 2,2-, 2,3-butanediyl, 1,1-, 1,2- or 1,3-(2-methyl-propanediyl), 1,1-ethenediyl, 1,2-ethenediyl, 1,1-, 1,2- or 1,3-propenediyl, ethinediyl, 1,3-propinediyl or a grouping -*O-CH₂-, -*O-CH₂-CH₂-, -*CH₂-O-, -*CH₂-O-CH₂-CH₂-, -*CH₂-S-, -*S-CH₂-, 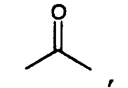 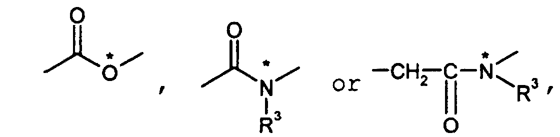 where the atom labelled by * is in each case attached to R¹,
R³ represents hydrogen or methyl,
R¹ represents hydrogen, or represents cyclopropyl, cyclobutyl, cyclopentyl, cyclohexyl, cyclopentenyl or cyclohexenyl, each of which is optionally mono- to trisubstituted by chlorine, methyl, ethyl, n- or i-propyl, or
R¹ represents phenyl which may be mono- to trisubstituted by identical or different substituents from the group consisting of fluorine, chlorine, bromine, nitro, carbamoyl, methyl, ethyl, n- or i-propyl, n-, i-, s- or t-butyl, methoxy, ethoxy, n- or i-propoxy, methylthio, ethylthio, n- or i-propylthio, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio or trifluoromethylsulphonyl, methylamino, ethylamino, n- or i-propylamino, dimethylamino, diethylamino, acetyl, propionyl, methoxycarbonyl, ethoxycarbonyl, hydroximinomethyl, hydroximinoethyl, methoximinomethyl, ethoximinomethyl, methoximinoethyl and ethoximinoethyl,
in each case doubly attached trimethylene (propane-1,3-diyl), methylenedioxy or ethylenedioxy, which is in each case optionally mono- to tetrasubstituted by identical or different substituents from the group consisting of fluorine, chlorine, methyl and trifluoromethyl,
cyclopropyl, cyclobutyl, cyclopentyl or cyclohexyl,
and/or
by phenyl, phenoxy, phenylthio, benzyl, benzyloxy, benzylthio, pyridyl, pyrimidinyl or thienyl, where these radicals may be mono-to trisubstituted by identical or different substituents from the group consisting of fluorine, chlorine, bromine, nitro, methyl, ethyl, n- or i-propyl, n-, i-, s- or t-butyl, methoxy, ethoxy, n- or i-propoxy, methylthio, ethylthio, n- or i-propylthio, trifluoromethyl, trifluoroethyl, difluoromethoxy, trifluoromethoxy, difluorochloromethoxy and trifluoroethoxy, or
R¹ represents thienyl, furyl or pyridyl, where these radicals may be mono- to trisubstituted by identical or different substituents from the group consisting of fluorine, chlorine, bromine, nitro, amino, hydroxyl, methyl, ethyl, n- or i-propyl, n-, i-, s- or t-butyl, methoxy, ethoxy, n- or i-propoxy, methylthio, ethylthio, n- or i-propylthio, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio or trifluoromethylsulphonyl, methylamino, ethylamino, nor i-propylamino, dimethylamino, diethylamino, acetyl, propionyl, methoxycarbonyl, ethoxycarbonyl, hydroximinomethyl, hydroximinoethyl, methoximinomethyl, ethoximinomethyl, methoximinoethyl or ethoximinoethyl, cyclopropyl, cyclobutyl, cyclopentyl and cyclohexyl,
and/or by
phenyl, phenoxy, phenylthio, benzyl, benzyloxy and/or benzylthio, where these aromatic radicals may be mono- to trisubstituted by identical or different substituents from the group consisting of fluorine, chlorine, bromine, nitro, methyl, ethyl, n- or i-propyl, n-, i-, s- or t-butyl, methoxy, ethoxy, n- or i-propoxy, methylthio, ethylthio, n- or i-propylthio, trifluoromethyl, trifluoroethyl, difluoromethoxy, trifluoromethoxy, difluorochloromethoxy, trifluoroethoxy, difluoromethylthio, difluorochloromethylthio and trifluoromethylthio, and
R² represents methyl, ethyl, n- or i-propyl, n-, i-, s- or t-butyl, dimethylamino or diethylamino, or
R² represents phenyl which may be mono- to trisubstituted by identical or different substituents from the group consisting of fluorine, chlorine, bromine, nitro, methyl, ethyl, methoxy, ethoxy, methylthio, ethylthio, trifluoromethyl, trifluoroethyl, difluoromethoxy, trifluoromethoxy, difluorochloromethoxy, trifluoroethoxy, difluoromethylthio, difluorochloromethylthio and trifluoromethylthio, or
R² represents furyl, thienyl, oxazolyl, isoxazolyl, pyrazolyl, imidazolyl, thiazolyl, isothiazolyl, oxadiazolyl, thiadiazolyl, pyridinyl, pyridazinyl, pyrazinyl, 1,2,3-triazinyl, 1,2,4-triazinyl, 1,3,5-triazinyl, 1,2,4-triazolonyl, pyrrolidinyl, piperidinyl or morpholinyl, where these radicals may be mono-, di- or trisubstituted by identical or different substituents from the group consisting of fluorine, chlorine, bromine, cyano, nitro, amino, hydroxyl, carbamoyl, methyl, ethyl, n- or i-propyl, n-, i-, s-, or t-butyl, cyclopropyl, methoxy, ethoxy, n- or i-propoxy, trifluoromethyl, trifluoroethyl, difluoromethoxy, trifluoromethoxy, acetyl, propionyl, methoxycarbonyl, ethoxycarbonyl, hydroxyiminomethyl, hydroxyiminoethyl, methoxyiminomethyl, ethoxyiminomethyl, methoxyiminoethyl and ethoxyiminoethyl,
for controlling undesirable microorganisms in crop protection and in the protection of materials.

2. Method for controlling undesirable microorganisms in crop protection and in the protection of materials, **characterized in that** sulphonyloxadiazolones of the formula (I) according to Claim 1 are applied to the microorganisms and/or their habitat.

3. Sulphonyloxadiazolones of the formula 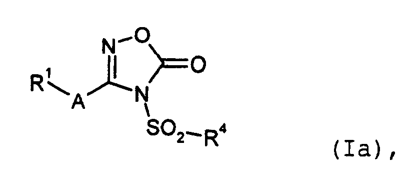 in which
A represents a single bond or represents methanediyl, 1,1-ethanediyl, 1,2-ethanediyl, 1,1-, 1,2-, 1,3- or 2,2-propanediyl, 1,1-, 1,2-, 1,3-, 1,4-, 2,2-, 2,3-butanediyl, 1,1-, 1,2- or 1,3-(2-methyl-propanediyl), 1,1-ethenediyl, 1,2-ethenediyl, 1,1-, 1,2- or 1,3-propenediyl, ethinediyl, 1,3-propinediyl or a grouping -*O-CH₂-, -*O-CH₂-CH₂-, -*CH₂-O-, -*CH₂-O-CH₂-CH₂-, -*CH₂-S-, -*S-CH₂-, 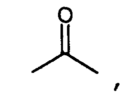 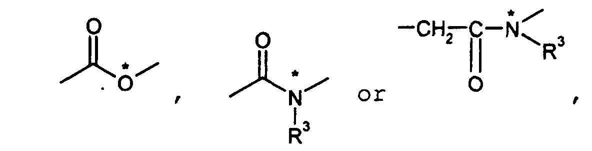 where the atom labelled by * is in each case attached to R¹,
R³ represents hydrogen or methyl,
R¹ represents hydrogen, or represents cyclopropyl, cyclobutyl, cyclopentyl, cyclohexyl, cyclopentenyl or cyclohexenyl, each of which is optionally mono- to trisubstituted by chlorine, methyl, ethyl, n- or i-propyl, or
R¹ represents phenyl which may be mono- to trisubstituted by identical or different substituents from the group consisting of fluorine, chlorine, bromine, nitro, carbamoyl, methyl, ethyl, n- or i-propyl, n-, i-, s- or t-butyl, methoxy, ethoxy, n- or i-propoxy, methylthio, ethylthio, n- or i-propylthio, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio or trifluoromethylsulphonyl, methylamino, ethylamino, n- or i-propylamino, dimethylamino, diethylamino, acetyl, propionyl, methoxycarbonyl, ethoxycarbonyl, hydroximinomethyl, hydroximinoethyl, methoximinomethyl, ethoximinomethyl, methoximinoethyl and ethoximinoethyl,
in each case doubly attached trimethylene (propane-1,3-diyl), methylenedioxy or ethylenedioxy, which is in each case optionally mono- to tetrasubstituted by identical or different substituents from. the group consisting of fluorine, chlorine, methyl and trifluoromethyl,
cyclopropyl, cyclobutyl, cyclopentyl or cyclohexyl,
and/or
by phenyl, phenoxy, phenylthio, benzyl, benzyloxy, benzylthio, pyridyl, pyrimidinyl or thienyl, where these radicals may be mono-to trisubstituted by identical or different substituents from the group consisting of fluorine, chlorine, bromine, nitro, methyl, ethyl, n- or i-propyl, n-, i-, s- or t-butyl, methoxy, ethoxy, n- or i-propoxy, methylthio, ethylthio, n- or i-propylthio, trifluoromethyl, trifluoroethyl, difluoromethoxy, trifluoromethoxy, difluorochloromethoxy and trifluoroethoxy, or
R¹ represents thienyl, furyl or pyridyl, where these radicals may be mono- to trisubstituted by identical or different substituents from the group consisting of fluorine, chlorine, bromine, nitro, amino, hydroxyl, methyl, ethyl, n- or i-propyl, n-, i-, s- or t-butyl, methoxy, ethoxy, n- or i-propoxy, methylthio, ethylthio, n- or i-propylthio, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio or trifluoromethylsulphonyl, methylamino, ethylamino, nor i-propylamino, dimethylamino, diethylamino, acetyl, propionyl, methoxycarbonyl, ethoxycarbonyl, hydroximinomethyl, hydroximinoethyl, methoximinomethyl, ethoximinomethyl, methoximinoethyl or ethoximinoethyl, cyclopropyl, cyclobutyl, cyclopentyl and cyclohexyl,
and/or by
phenyl, phenoxy, phenylthio, benzyl, benzyloxy and/or benzylthio, where these aromatic radicals may be mono- to trisubstituted by identical or different substituents from the group consisting of fluorine, chlorine, bromine, nitro, methyl, ethyl, n- or i-propyl, n-, i-, s- or t-butyl, methoxy, ethoxy, n- or i-propoxy, methylthio, ethylthio, n- or i-propylthio, trifluoromethyl, trifluoroethyl, difluoromethoxy, trifluoromethoxy, difluorochloromethoxy, trifluoroethoxy, difluoromethylthio, difluorochloromethylthio and trifluoromethylthio, and
R⁴ represents dimethylamino or diethylamino, or
R⁴ represents nitro-substituted phenyl which may additionally be mono- or disubstituted by identical or different substituents from the group consising of fluorine, chlorine, bromine, nitro, methyl, ethyl, methoxy, ethoxy, methylthio, ethylthio, trifluoromethyl, trifluoroethyl, difluoromethoxy, trifluoromethoxy, difluorochloromethoxy, trifluoroethoxy, difluoromethylthio, difluorochloromethylthio and trifluoromethylthio, or
R⁴ represents furyl, thienyl, oxazolyl, isoxazolyl, pyrazolyl, imidazolyl, thiazolyl, isothiazolyl, oxadiazolyl, thiadiazolyl, pyridinyl, pyridazinyl, pyrazinyl, 1,2,3-triazinyl, 1,2,4-triazinyl, 1,3,5-triazinyl, 1,2,4-triazolonyl, pyrrolidinyl, piperidinyl or morpholinyl, where these radicals may be mono-, di- or trisubstituted by identical or different substituents from the group consisting of fluorine, chlorine, bromine, cyano, nitro, amino, hydroxyl, carbamoyl, methyl, ethyl, n- or i-propyl, n-, i-, s-, or t-butyl, cyclopropyl, methoxy, ethoxy, n- or i-propoxy, trifluoromethyl, trifluoroethyl, difluoromethoxy, trifluoromethoxy, acetyl, propionyl, methoxycarbonyl, ethoxycarbonyl, hydroxyiminomethyl, hydroxyiminoethyl, methoxyiminomethyl, ethoxyiminomethyl, methoxyiminoethyl and ethoxyiminoethyl.

4. Process for preparing sulphonyloxadiazolones of the formula (Ia) according to Claim 3,
**characterized in that**
oxadiazolones of the formula 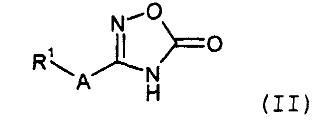 in which
A and R¹ are as defined above,
are reacted with sulphonyl halides of the formula
R⁴-SO₂-X (III)
in which
R⁴ is as defined above and
X represents chlorine,
if appropriate in the presence of an acid binder and if appropriate in the presence of a diluent.

5. Microbicidal compositions, **characterized in that** they comprise at least one sulphonyloxadiazolone of the formula (Ia) according to Claim 3, in addition to extenders and/or surfactants.

6. Process for preparing microbicidal compositions, **characterized in that** sulphonyloxadiazolones of the formula (Ia) according to Claim 3 are mixed with extenders and/or surfactants.

7. Sulphonyloxadiazolone according to Claim 3, **characterized by** the formula

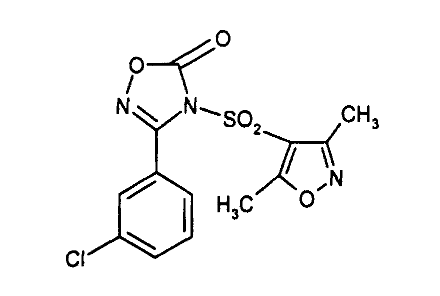

8. Sulphonyloxadiazolone according to Claim 3, **characterized by** the formula

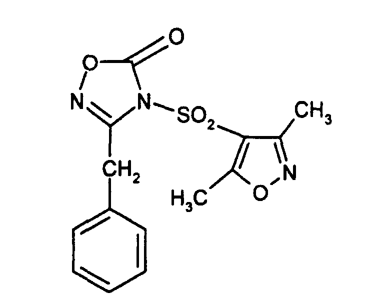

9. Sulphonyloxadiazolone according to Claim 3, **characterized by** the formula

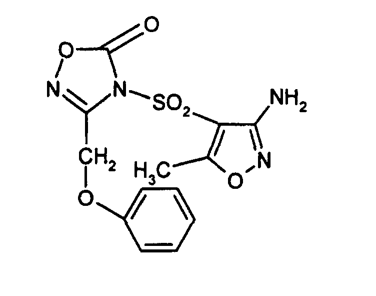

## Revendications

1. Utilisation de sulfonyloxadiazolones de formule 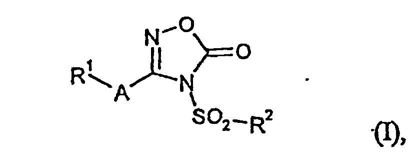 dans laquelle
A représente une liaison simple ou un reste méthanediyle, 1,1-éthanediyle, 1,2-éthanediyle, 1,1-, 1,2-, 1,3- ou 2,2-propanediyle, 1,1-, 1,2-, 1,3-, 1,4-, 2,2-, 2,3-butanediyle, 1,1-, 1,2- ou 1,3-(2-méthylpropanediyle), 1,1-éthènediyle, 1,2-éthènediyle, 1,1-, 1,2- ou 1,3-propènediyle, éthynediyle, 1,3-propynediyle ou un groupement -*O-CH₂-, -*O-CH₂-CH₂-, -*CH₂-O-, -*CH₂-O-CH₂-CH₂-, -*CH₂-S-, -*S-CH₂-, 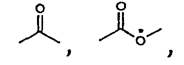 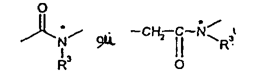 l'atome marqué d'un astérisque étant lié dans chaque cas à R¹,
R³ représente un atome d'hydrogène ou un reste méthyle,
R¹ représente un atome d'hydrogène ou un reste cyclopropyle, cyclobutyle, cyclopentyle, cyclohexyle, cyclopentényle ou cyclohexényle dont chacun porte éventuellement un à trois substituants chloro, méthyle, éthyle, n-propyle ou isopropyle,
ou bien
est un reste phényle qui peut porter un à trois substituants, identiques ou différents, fluoro, chloro, bromo, nitro, carbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio ou trifluorométhylsulfonyle, méthylamino, éthylamino, n-propylamino, isopropylamino, diméthylamino, diéthylamino, acétyle, propionyle, méthoxycarbonyle, éthoxycarbonyle, hydroximinométhyle, hydroximino-éthyle, méthoximinométhyle, éthoximinométhyle, méthoximinoéthyle ou éthoximino-éthyle,
un reste triméthylène (propane-1,3-diyle), méthylènedioxy ou éthylènedioxy dont chacun a deux liaisons et porte éventuellement un à quatre substituants, identiques ou différents, fluoro, chloro, méthyle ou trifluorométhyle,
un reste cyclopropyle, cyclobutyle, cyclopentyle ou cyclohexyle,
et/ou
par un reste phényle, phénoxy, phénylthio, benzyle, benzyloxy, benzylthio, pyridyle, pyrimidinyle ou thiényle, ces restes pouvant porter un à trois substituants, identiques ou différents, fluoro, chloro, bromo, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, trifluorométhyle, trifluoréthyle, difluorométhoxy, trifluorométhoxy, difluorochlorométhoxy ou trifluoréthoxy,
ou bien
R¹ est un reste thiényle, furyle ou pyridyle, ces restes pouvant porter un à trois substituants, identiques ou différents, fluoro, chloro, bromo, nitro, amino, hydroxy, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfonyle, méthylamino, éthylamino, n-propylamino, isopropylamino, diméthylamino, diéthylamino, acétyle, propionyle, méthoxycarbonyle, éthoxycarbonyle, hydroximinométhyle, hydroximino-éthyle, méthoximinométhyle, éthoximinométhyle, méthoximinoéthyle, éthoximino-éthyle, cyclopropyle, cyclobutyle, cyclopentyle et/ou cyclohexyle,
et/ou par
un reste phényle, phénoxy, phénylthio, benzyle, benzyloxy et/ou benzylthio, ces restes aromatiques pouvant porter un à trois substituants, identiques ou différents, fluoro, chloro, bromo, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, trifluorométhyle, trifluoréthyle, difluorométhoxy, trifluorométhoxy, difluorochlorométhoxy, trifluoréthoxy, difluorométhylthio, difluorochlorométhylthio et/ou trifluorométhylthio,
et
R² est un reste méthyle, éthyle, n-propyle, isopropyle, n-butyle, isobutyle, sec.-butyle, tertio-butyle, diméthylamino ou diéthylamino,
ou bien
R² est un reste phényle qui peut porter un à trois substituants, identiques ou différents, fluoro, chloro, bromo, nitro, méthyle, éthyle, méthoxy, éthoxy, méthylthio, éthylthio, trifluorométhyle, trifluoréthyle, difluorométhoxy, trifluorométhoxy, difluorochlorométhoxy, trifluoréthoxy, difluorométhylthio, difluorochlorométhylthio ou trifluorométhylthio,
ou bien
R² est un reste furyle, thiényle, oxazolyle, isoxazolyle, pyrazolyle, imidazolyle, thiazolyle, isothiazolyle, oxadiazolyle, thiadiazolyle, pyridinyle, pyridazinyle, pyrazinyle, 1,2,3-triazinyle, 1,2,4-triazinyle, 1,3,5-triazinyle, 1,2,4-triazolonyle, pyrrolidinyle, pipéridinyle ou morpholinyle, ces restes pouvant porter un, deux ou trois substituants, identiques ou différents, fluoro, chloro, bromo, cyano, nitro, amino, hydroxy, carbamoyle, méthyle, éthyle, n-propyle, isopropyle, n-butyle, isobutyle, sec.-butyle, tertio-butyle, cyclopropyle, méthoxy, éthoxy, n-propoxy, isopropoxy, trifluorométhyle, trifluoréthyle, difluorométhoxy, trifluorométhoxy, acétyle, propionyle, méthoxycarbonyle, éthoxycarbonyle, hydroxyiminométhyle, hydroxyimino-éthyle, méthoxyiminométhyle, éthoxyiminométhyle, méthoxyimino-éthyle et/ou éthoxyimino-éthyle,
pour combattre des micro-organismes indésirables dans la protection des plantes et dans la protection des matériaux.

2. Procédé pour combattre des micro-organismes indésirables dans la protection des plantes et dans la protection des matériaux, **caractérisé en ce qu'**on épand des sulfonyloxadiazolones de formule (I) suivant la revendication 1 sur les micro-organismes et/ou sur leur milieu.

3. Sulfonyloxadiazolones de formule 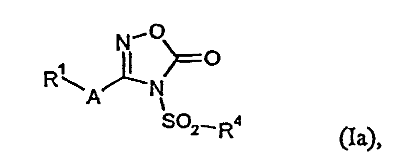 dans laquelle
A représente une liaison simple ou un reste méthanediyle, 1,1-éthanediyle, 1,2-éthanediyle, 1,1-, 1,2-, 1,3- ou 2,2-propanediyle, 1,1-, 1,2-, 1,3-, 1,4-, 2,2- ou 2,3-butanediyle, 1,1-, 1,2- ou 1,3-(2-méthylpropanediyle), 1,1-éthènediyle, 1,2-éthènediyle, 1,1-, 1,2- ou 1,3-propènediyle, éthynediyle, 1,3-propynediyle ou un groupement -*O-CH₂-, -*O-CH₂-CH₂-, -*CH₂-O-, -*CH₂-O-CH₂-CH₂-, -*CH₂-S-, -*S-CH₂-, 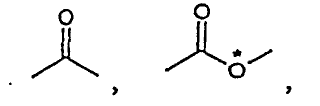 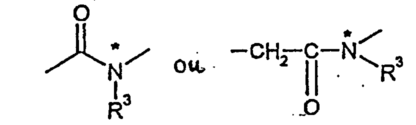 l'atome portant un astérisque étant lié dans chaque cas à R¹,
R³ représente l'atome d'hydrogène ou un reste méthyle,
R¹ représente l'atome d'hydrogène ou un reste cyclopropyle, cyclobutyle, cyclopentyle, cyclohexyle, cyclopentényle ou cyclohexényle portant chacun, le cas échéant, un à trois substituants chloro, méthyle, éthyle, n-propyle ou isopropyle,
ou bien
R¹ est un reste phényle qui peut porter un à trois substituants, identiques ou différents, fluoro, chloro, bromo, nitro, carbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio ou trifluorométhylsulfonyle, méthylamino, éthylamino, n-propylamino, isopropylamino, diméthylamino, diéthylamino, acétyle, propionyle, méthoxycarbonyle, éthoxycarbonyle, hydroximinométhyle, hydroximino-éthyle, méthoximinoméhtyle, éthoximinométhyle, méthoximinoéthyle ou éthoximino-éthyle,
un reste triméthylène (propane-1,3-diyle), méthylènedioxy ou éthylènedioxy ayant chacun deux liaisons et portant, le cas échéant, un à quatre substituants, identiques ou différents, fluoro, chloro, méthyle ou trifluorométhyle,
cyclopropyle, cyclobutyle, cyclopentyle ou cyclohexyle,
et/ou
par un reste phényle, phénoxy, phénylthio, benzyle, benzyloxy, benzylthio, pyridyle, pyrimidinyle ou thiényle, ces restes pouvant porter un à trois substituants, identiques ou différents, fluoro, chloro, bromo, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, trifluorométhyle, trifluoréthyle, difluorométhoxy, trifluorométhoxy, difluorochlorométhoxy ou trifluoréthoxy,
ou bien
R¹ est un reste thiényle, furyle ou pyridyle, ces restes pouvant porter un à trois substituants, identiques ou différents, fluoro, chloro, bromo, nitro, amino, hydroxy, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio ou trifluorométhylsulfonyle, méthylamino, éthylamino, n-propylamino, isopropylamino, diméthylamino, diéthylamino, acétyle, propionyle, méthoxycarbonyle, éthoxycarbonyle, hydroximinométhyle, hydroximino-éthyle, méthoximinométhyle, éthoximinométhyle, méthoximinoéthyle, éthoximino-éthyle, cyclopropyle, cyclobutyle, cyclopentyle et/ou cyclohexyle,
et/ou par
un reste phényle, phénoxy, phénylthio, benzyle, benzyloxy et/ou benzylthio, ces restes aromatiques pouvant porter un à trois substituants, identiques ou différents, fluoro, chloro, bromo, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, trifluorométhyle, trifluoréthyle, difluorométhoxy, trifluorométhoxy, difluorochlorométhoxy, trifluoréthoxy, difluorométhylthio, difluorochlorométhylthio et/ou trifluorométhylthio,
et
R⁴ est un reste diméthylamino ou diéthylamino,
ou bien
R⁴ est un reste phényle portant un substituant nitro, qui peut encore porter en outre un ou deux substituants, identiques ou différents, fluoro, chloro, bromo, nitro, méthyle, éthyle, méthoxy, éthoxy, méthylthio, éthylthio, trifluorométhyle, trifluoréthyle, difluorométhoxy, trifluorométhoxy, difluorochlorométhoxy, trifluoréthoxy, difluorométhylthio, difluorochlorométhylthio ou trifluorométhylthio,
ou bien
R⁴ est un reste furyle, thiényle, oxazolyle, isoxazolyle, pyrazolyle, imidazolyle, thiazolyle, isothiazolyle, oxadiazolyle, thiadiazolyle, pyridinyle, pyridazinyle, pyrazinyle, 1,2,3-triazinyle, 1,2,4-triazinyle, 1,3,5-triazinyle, 1,2,4-triazolonyle, pyrrolidinyle, pipéridinyle ou morpholinyle, ces restes pouvant porter un, deux ou trois substituants, identiques ou différents, fluoro, chloro, bromo, cyano, nitro, amino, hydroxy, carbamoyle, méthyle, éthyle, n-propyle, isopropyle, n-butyle, isobutyle, sec.-butyle, tertio-butyle, cyclopropyle, méthoxy, éthoxy, n-propoxy, isopropoxy, trifluorométhyle, trifluoréthyle, difluorométhoxy, trifluorométhoxy, acétyle, propionyle, méthoxycarbonyle, éthoxycarbonyle, hydroxyiminométhyle, hydroxyimino-éthyle, méthoxyiminométhyle, éthoxyiminométhyle, méthoxyimino-éthyle et/ou éthoxyimino-éthyle.

4. Procédé de production de sulfonyloxadiazolones de formule (Ia) suivant la revendication 3, **caractérisé en ce qu'**on fait réagir des oxadiazolones de formule 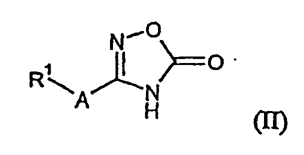 dans laquelle
A et R¹ ont les définitions indiquées ci-dessus,
avec des halogénures d'acide sulfonique de formule
R⁴-SO₂-X (III)
dans laquelle
R⁴ a la définition indiquée ci-dessus et
X représente le chlore,
le cas échéant en présence d'un accepteur d'acide et en présence éventuelle d'un diluant.

5. Compositions microbicides, **caractérisées par** une teneur en au moins une sulfonyloxadiazolone de formule (Ia) suivant la revendication 3, à côté de diluants et/ou de substances tensio-actives.

6. Procédé de préparation de compositions microbicides, **caractérisé en ce qu'**on mélange des sulfonyloxadiazolones de formule (Ia) suivant la revendication 3, avec des diluants et/ou des agents tensio-actifs.

7. Sulfonyloxadiazolone suivant la revendication 3, **caractérisée par** la formule

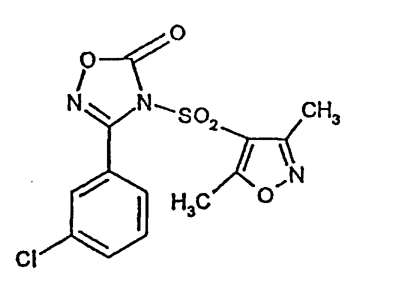

8. Sulfonyloxadiazolone suivant la revendication 3, **caractérisée par** la formule

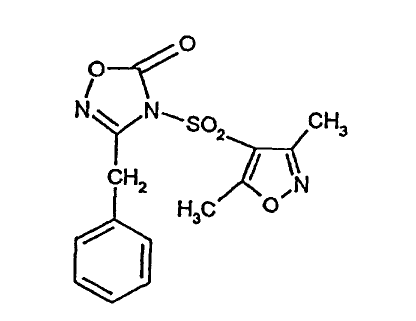

9. Sulfonyloxadiazolone suivant la revendication 3, **caractérisée par** la formule
